(19) Europäisches Patentamt
European Patent Office
Office européen des brevets

(11) EP 0 559 538 B1

(12) **FASCICULE DE BREVET EUROPEEN**

(45) Date de publication et mention de la délivrance du brevet:
**17.05.2000 Bulletin 2000/20**

(51) Int. Cl.[7]: **C07D 211/14**, C07D 211/48, C07D 211/52, C07D 211/58, C07D 401/06, C07D 409/12, A61K 31/445

(21) Numéro de dépôt: **93400513.3**

(22) Date de dépôt: **01.03.1993**

(54) **Sels quaternaires de pipéridines 4-substitués, leur préparation et compositions pharmaceutiques les contenant**

Quartäre Salze 4-substituierter Piperidine, ihre Herstellung sowie diese enthaltende Arzneimittel

Quaternary salts of 4-substituted piperidines, their preparation and pharmaceutical compositions containing them

(84) Etats contractants désignés:
**AT BE CH DE DK ES FR GB GR IE IT LI LU NL PT SE**

(30) Priorité: **03.03.1992 FR 9202542**
**29.10.1992 FR 9212941**

(43) Date de publication de la demande:
**08.09.1993 Bulletin 1993/36**

(73) Titulaire: **Sanofi-Synthélabo**
**75013 Paris (FR)**

(72) Inventeurs:
- **Emonds-Alt, Xavier**
  **F-34980 Combaillaux (FR)**
- **Proietto, Vincenzo**
  **F-34680 Saint Georges d'Orques (FR)**
- **Van Broeck, Didier**
  **F-34570 Murviel les Montpellier (FR)**
- **Breliere, Jean-Claude**
  **F-34090 Montpellier (FR)**

(74) Mandataire:
**Gillard, Marie-Louise et al**
**Cabinet Beau de Loménie**
**158, rue de l'Université**
**75340 Paris Cédex 07 (FR)**

(56) Documents cités:
**EP-A- 0 288 352**
**EP-A- 0 428 434**
**EP-A- 0 474 561**

- **JOURNAL OF MEDICINAL CHEMISTRY. vol. 14, no. 9, 1971, Washington, US, pages 896-897**
- **JOURNAL OF MEDICINAL CHEMISTRY. vol. 13, no. 4, 1970, Washington, US, pages 747-748**
- **CHEMICAL ABSTRACTS, vol. 118, abrégé-no. 32786, Columbus, Ohio, US; ADVENIER C. et al.: "Effects on the isolated human bronchus of SR 48968, a potent and selective nonpeptide antagonist of the neurokinin A (NK2) receptors"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.7 (HRS)/3.6

## Description

La présente invention a pour objet de nouveaux sels d'ammonium quaternaires de dérivés aromatiques substitués par un groupement amino et par diverses fonctions esters, amines ou amides, ainsi que leurs énantiomères.

La présente invention concerne également le procédé de préparation des composés et l'utilisation des composés selon l'invention dans des compositions à usage thérapeutique et plus particulièrement dans les phénomènes pathologiques qui impliquent le système des neurokinines comme : la douleur (D. Regoli et al., Life Sciences, 1987, *40*, 109-117), l'allergie et l'inflammation (J.E. Morlay et al., Life Sciences, 1987, *41*, 527-544), l'insuffisance circulatoire (J. Losay et al., 1977, Substance P, Von Euler, U.S. and Pernow ed., 287-293, Raven Press, New York), les troubles gastrointestinaux (D. Regoli et al., Trends Pharmacol. Sci., 1985, *6*, 481-484), les troubles respiratoires (J. Mizrahi et al., Pharmacology, 1982, *25*, 39-50).

Des ligands endogènes aux récepteurs des neurokinines ont été décrits, telles la substance P (SP), la neurokinine A (NKA) (S.J. Bailey et al., 1983, Substance P. P. Skrabanck ed., 16-17 Boole Press, Dublin) et la neurokinine B (NKB) (S.P. Watson, Life Sciences, 1983, *25*, 797-808).

Les récepteurs aux neurokinines ont été reconnus sur de nombreuses préparations et sont actuellement classés en trois types : $NK_1$, $NK_2$ et $NK_3$. Alors que la plupart des préparations étudiées jusqu'à maintenant, présentent plusieurs types de récepteurs, tel l'iléon de cobaye ($NK_1$, $NK_2$ et $NK_3$), certaines d'entre elles n'en possèderaient qu'un seul, telles l'artère carotide de chien ($NK_1$), l'artère pulmonaire de lapin dépourvue d'endothélium ($NK_2$) et la veine porte de rat ($NK_3$) (D. Regoli et al., Trends Pharmacol. Sci., 1988, *38*, 1-15).

Une caractérisation plus précise des différents récepteurs est rendue possible par la synthèse récente d'agonistes sélectifs. Ainsi, la [$Sar^9$, Met-$(O_2)^{11}$] SP, la [$Nle^{10}$] $NK_{4-10}$, et la [Me $Phe^7$]-NKB présenteraient une sélectivité respective pour les récepteurs $NK_1$, $NK_2$ et $NK_3$ (cf D. Regoli, 1988 et 1989 précédemment cité).

Le récepteur $NK_2$ et la neurokinine A sont, par exemple, impliqués dans les inflammations neurogéniques des voies respiratoires (P.J. Barnes, Arch. Int. Pharmacodyn., 1990, *303*, 67-82 et G.F. Joos et al., Arch. Int. Pharmacodyn., 1990, *303*, 132-146).

La demande de brevet EP-A-336230 décrit des dérivés peptidiques antagonistes de la substance P et de la neurokinine A utiles pour le traitement et la prévention de l'asthme.

Les demandes de brevet internationales WO 90/05525, WO 90/05729, WO 91/09844, WO 91/18899 et européennes EP-A-436334, EP-A-429466 et EP-A-430771 décrivent des antagonistes de la Substance P.

On a maintenant trouvé que certains sels d'ammonium quaternaires de composés aromatiques aminés possèdent des propriétés pharmacologiques intéressantes, en tant qu'antagonistes des récepteurs des neurokinines et sont notamment utiles pour le traitement de toute pathologie substance P et neurokinine dépendantes.

Snider et al. (Sciences, 1991, *251*, 435-437) ont montré, en utilisant un antagoniste non peptidique de la Substance P, le CP 96,345, que l'affinité d'un tel agoniste pour les récepteurs de la Substance P était très fortement fonction de l'espèce animale. Ainsi, d'après ces auteurs, le CP 96,345 a un Ki égal à 240 nM pour le récepteur rat (liaison 3H-SP sur les membranes de cortex de rat) mais a un Ki égal à 3 nM pour le récepteur boeuf (liaison 3H-SP sur la membrane de caudate de boeuf). D'autres auteurs ont montré que le CP 96,345 a un Ki égal à 0,4 nM dans des essais de liaison de la Substance P sur des cellules lymphoblastiques humaines IM9, U373 MG. (Gitter et al., Eur. J. Pharmacol., 1991, *197*, 237-238).

Plus particulièrement on a trouvé que certains sels d'ammonium quaternaires, dont notamment ceux des composés revendiqués dans la demande EP-A-428434, EP 474561 ont une affinité augmentée pour les récepteurs de la Substance P de cellules humaines de la lignée IM9.

On a également trouvé que ces sels d'ammonium quaternaires montrent de façon tout à fait surprenante un accroissement important, par rapport aux amines tertiaires correspondantes, de l'affinité pour les récepteurs de la Substance P sur des cellules humaines (IM9) sans pour autant que leur affinité pour les récepteurs de la Substance P du cortex de rat soit sensiblement diminuée.

Enfin, on a trouvé que ces sels quaternaires, contrairement à ce que l'on pouvait attendre, sont actifs *in vivo* et par la voie orale et que cette activité peut s'exercer également sur le système nerveux central.

Ainsi, selon un de ses aspects, la présente invention concerne les sels d'ammonium quaternaires de composés aromatiques aminés diversement substitués de formule :

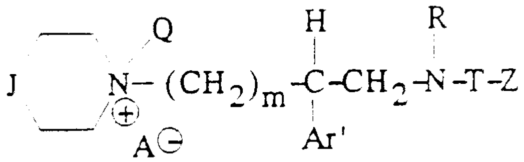 (I)

dans laquelle :

- J représente
  * - soit un groupe

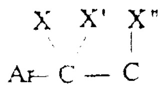

dans lequel

. Ar représente un phényle non substitué ou substitué une ou plusieurs fois par un substituant choisi parmi : l'hydrogène, un atome d'halogène, un hydroxyle, un alcoxy en $C_1$-$C_3$, un alkyle en $C_1$-$C_3$, un trifluorométhyle, lesdits substituants étant identiques ou différents ; un groupe cycloalkyle en $C_3$-$C_7$ ; un groupe pyridyle ou un groupe thiényle ;
. X représente l'hydrogène ;
. X' représente l'hydrogène ou est réuni à X" ci-dessous pour former une liaison carbone-carbone ou bien X et X' ensemble forment un groupe oxo ;
. X" est l'hydrogène ou avec X' forme une liaison carbone-carbone ;

  * - soit un groupe

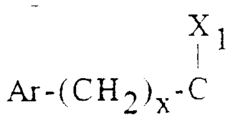

dans lequel :

. Ar est tel que défini ci-dessus ;
. x est zéro ou un ;
. $X_1$ représente l'hydrogène, uniquement lorsque x est zéro ; un hydroxyle; un alcoxy en $C_1$-$C_4$ ; un acyloxy en $C_1$-$C_4$ ; un carboxy ; un carbalcoxy en $C_1$-$C_4$ ; un cyano ; un groupe -NH-CO-Alk dans lequel Alk représente un alkyle en $C_1$-$C_6$ ; un groupe mercapto ; ou un groupe alkylthio en $C_1$-$C_4$ ;
ou bien $X_1$ forme avec l'atome de carbone auquel il est lié et avec l'atome de carbone voisin dans la pipéridine une double liaison ;

- Q représente un groupe alkyle en $C_1$-$C_6$ ou un groupe benzyle ;
- $A^{\ominus}$ représente un anion choisi par exemple parmi les anions chlorure, bromure, iodure, acétate, méthanesulfonate, paratoluènesulfonate ;
- m est 2 ou 3 ;
- Ar' représente un phényle non substitué ou substitué une ou plusieurs fois par un substituant choisi parmi ; l'hydrogène, un atome d'halogène, de préférence un atome de chlore ou de fluor, un trifluorométhyle, un alcoxy en $C_1$-$C_4$, un alkyle en $C_1$-$C_4$, lesdits substituants étant identiques ou différents ; un thiényle ; un benzothiényle ; un naphtyle ; un indolyle éventuellement N-substitué par un alkyle en $C_1$-$C_4$ ;

- R représente l'hydrogène ou un alkyle en $C_1$-$C_6$ ;
- T représente un groupe choisi parmi :

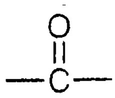

et

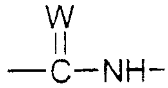

W étant un atome d'oxygène ou de soufre, et

- Z représente un naphtyle, un benzyle, un α-hydroxybenzyle, un phényle, éventuellement substitués par un substituant choisi parmi le chlore ou le fluor, un alkyle en $C_1$-$C_4$ ou un alcoxy en $C_1$-$C_4$.

Dans la présente description les groupes alkyle ou les groupes alcoxy sont droits ou ramifiés, les substituants Q sont soit en position axiale soit en position équatoriale.

Les amines tertiaires, précurseurs des amines quaternaires (I) selon l'invention seront nommées ci-dessous composés de formule (Ia).

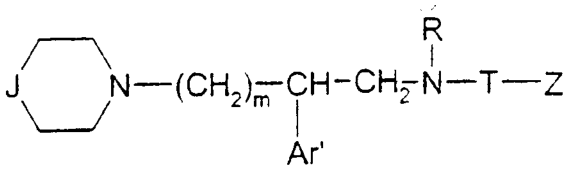

(Ia)

Les composés de formule (Ia) dans laquelle J est un groupe :

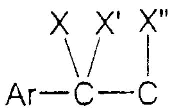

sont décrits dans EP-A2-0 428 434.

Les composés de formule (Ia) dans laquelle J est un groupe :

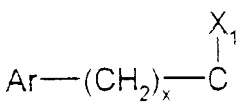

dans lequel x est un sont décrits dans EP-A1-0 474 561.

En revanche, les composés de formule (Ia) dans laquelle m, Ar', R, T et Z sont tels que définis ci-dessus et J est un groupe

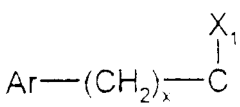

où Ar est tel que défini ci-dessus, x est zéro et $X_1$ est l'hydrogène, ainsi que leurs sels, de préférence les sels pharmaceutiquement acceptables, sont nouveaux, et font partie de l'invention.

Plus particulièrement, les précurseurs de formule (Ia'),

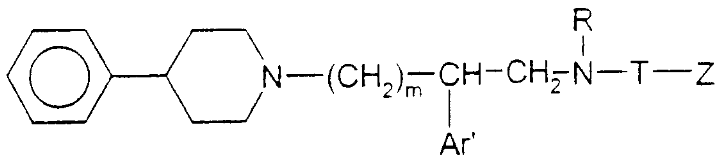 (Ia')

dans laquelle m, Ar', R, T et Z sont tels que définis ci-dessus, ainsi que leurs sels d'addition acides, de préférence pharmaceutiquement acceptables, sont préférés.

D'autres précurseurs, ceux de la formule (Ia) dans laquelle m, Ar', R et J sont tels que définis ci-dessus, T est -C(O)- et Z représente un $\alpha$-hydroxybenzyle dont le coupe aromatique est non substitué ou substitué une ou plusieurs fois par un chlore, un fluor, un alkyle en $C_1$-$C_4$, un alcoxy en $C_1$-$C_4$, ainsi que leurs sels, de préférence pharmaceutiquement acceptables, sont nouveaux et font partie de l'invention. Plus particulièrement, les composés de formules (Ia"),

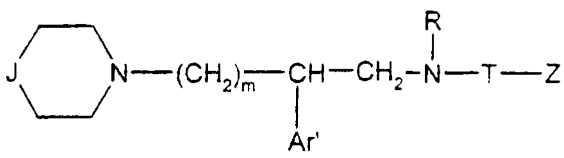 (Ia")

dans laquelle J, m, Ar', R, T et Z sont tels que définis ci-dessus, ainsi que leurs sels d'addition acide, de préférence pharmaceutiquement acceptables, sont préférés.

Les sels des composés de formule (Ia), (Ia') et (Ia") sont ceux formés avec des acides minéraux ou organiques qui permettent une séparation ou une cristallisation convenable des composés de formule (Ia), (Ia') et (Ia"), tels que l'acide picrique ou l'acide oxalique ou un acide optiquement actif, par exemple un acide mandélique ou camphosulfonique, ou un sel pharmaceutiquement acceptable tel que le chlorhydrate, le bromhydrate, le sulfate, l'hydrogénosulfate, le dihydrogénophosphate, le méthanesulfonate, le méthylsulfate, le maléate, le fumarate, le naphtalène-2 sulfonate, le glycolate, le gluconate, le citrate, l'iséthionate.

Dans la formule (I), T représente de préférence une groupe carbonyle.

Le substituant Ar' est de préférence un groupe phényle, avantageusement substitué par deux atomes de chlore, plus particulièrement dans les positions 3 et 4.

Dans la même formule (I), m est de préférence 2.

Les substituant J de la formule (I) est avantageusement un groupe

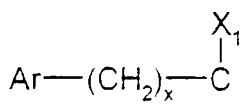

dans lequel Ar est phényle, x est zéro et $X_1$ est l'hydrogène ou un groupe acétylamino. Dans des composés avantageux, Z représente naphtyle, benzyle, phényle éventuellement substitués dans le cycle aromatique par un substituant choisi parmi chlore ou fluor, alkyle ou alcoxy en $C_1$-$C_4$. De préférence, Z est un groupe benzyle substitué par un groupe isopropoxy, avantageusement en position 3 ou un groupe 1-naphtyle substitué par un atome d'halogène, avantageusement par un fluor en position 4. Dans ces composés avantageux, plus particulièrement, le groupe T est un carbonyle, le groupe R est l'hydrogène ou un groupe méthyle et Ar' est 3,4-dichlorophényle.

Des composés quaternaires particulièrement préférés selon la présente invention sont ceux de formule (I) dans laquelle à la fois:

- Z est un groupe 3-isopropoxybenzyle ou 4-fluoro-1-naphtyle ;
- T est un groupe carbonyle ;
- R et Q sont tous les deux méthyle ;
- Ar' est 3,4-dichlorophényle ;
- m est 2 ;
- $A^{\ominus}$ est un anion pharmaceutiquement acceptable, le chlorure ou le méthanesulfonate étant particulièrement préférés.

Parmi ces produits, ceux représentés par la formule:

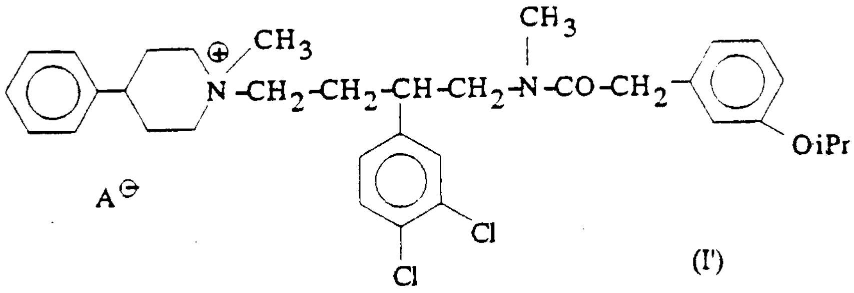

(I')

dans laquelle iPr représente isopropyle et A $^{\ominus}$ est tel que défini ci-dessus, notamment l'ion méthanesulfonate, iodure ou chlorure, sont des puissants antagonistes de la substance P, le composé ayant le groupe méthyle en position axiale étant particulièrement préféré.

D'autres composés de formule :

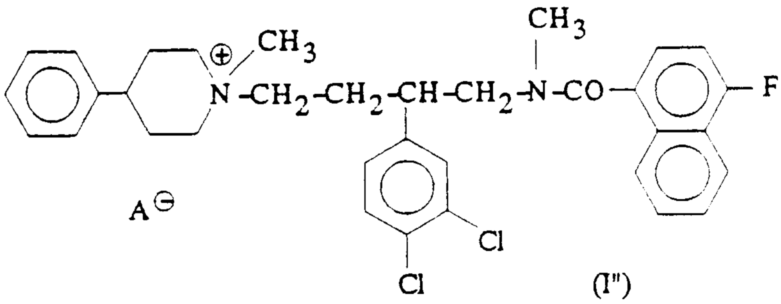

(I")

dans laquelle A $^{\ominus}$ est un anion pharmaceutiquement acceptable, notamment le méthanesulfonate, l'iodure et le chlorure, sont extrêmement intéressants.

Les composés de formule (I') et (I") sont extrêmement puissants et montrent une grande affinité pour les récepteurs de la neurokinine 2 et/ou 1. Ils constituent donc l'aspect préféré de la présente invention.

Le N-méthyl-N-[4-(4-phényl-1-pipéridinyl)-2-(3,4-dichlorophényl)butyl]-3-iso-propoxyphénylacétamide de formule :

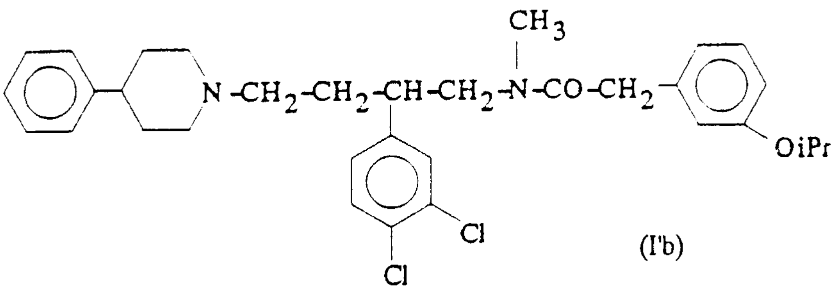

(I'b)

est un intermédiaire clé permettant la préparation du composé (I') et il est lui-même pourvu d'une très grande affinité pour les récepteurs de la neurokinine. Ce composé, ses énantiomères et ses sels d'addition acides, dont les sels d'addition acides pharmaceutiquement acceptables sont particulièrement avantageux, constituent un autre aspect préféré de la présente invention.

Selon un autre de ses aspects, la présente invention concerne un procédé pour la préparation des composés de formule (I), caractérisé en ce que :

on traite un composé de formule (Ia) par un excès d'agent alkylant de formule :

A - Q

dans lequel A représente un groupe susceptible de former un anion et est tel que défini précédemment pour (I), de préférence un chlorure ou un iodure et Q est tel que défini précédemment pour (I) et on chauffe le mélange réactionnel dans un solvant organique par exemple choisi parmi le dichlorométhane, le chloroforme, l'acétone ou l'acétonitrile, à une température comprise entre la température ambiante et le reflux pendant une à plusieurs heures pour obtenir après traitement selon les méthodes habituelles et après déprotection éventuelle, un mélange des conformères axiaux et équatoriaux des sels d'ammonium quaternaires.

Dans le cas où A $^{\ominus}$ représente un anion non pharmacologiquement acceptable ou, de toute façon, lorsqu'on souhaite obtenir un anion différent de celui obtenu à la fin de la réaction de quaternarisation, on peut échanger cet anion par un autre anion, par exemple un chlorure par action de l'acide approprié, éventuellement par élution du composé (I) sur une résine échangeuse d'ion, comme la résine Amberlite IRA68® ou DuoliteA375®.

Les conformères sont séparés selon les méthodes habituelles, par exemple par chromatographie ou par recristallisation.

Chacun des conformères axiaux ou équatoriaux des composés (I) sous forme de racémiques ou sous forme d'énantiomères R ou S optiquement purs fait partie de l'invention.

Les composés (Ia) sont préparés par un procédé selon lequel :

- a) on traite une amine libre de formule :

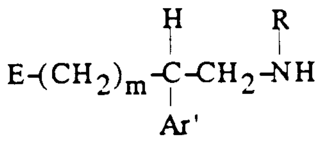

(II)

dans laquelle m, Ar' et R sont tels que définis précédemment et E représente un groupe O-protégé tel que par exemple 2-tétrahydropyranyloxy ou un groupe :

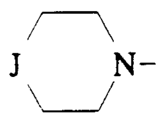

dans lequel J est tel que défini précédemment étant entendu que :

lorsque J représente le groupe

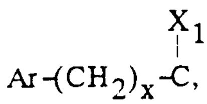

où $X_1$ est un hydroxyle, cet hydroxyle est protégé ;

- soit avec un dérivé fonctionnel d'un acide de formule :

$$HO - CO - Z \quad (III)$$

dans laquelle Z est tel que défini précédemment étant entendu que lorsque le groupe Z contient un hydroxyle, cet hydroxyle est protégé, lorsqu'on doit préparer un composé de formule (Ia) où T est -CO-,
- soit avec un iso(thio)cyanate de formule :

$$W = C = N - Z \quad (III')$$

dans laquelle W et Z sont tels que définis précédemment, lorsqu'on doit préparer un composé de formule (Ia) où T est -C(W)-NH-, pour former le composé de formule :

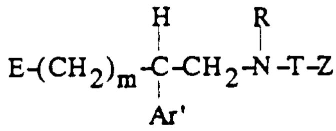 (IV)

- b) puis, lorsque E représente le 2-tétrahydropyranyloxy, on élimine le groupe 2-tétrahydropyranyle par action d'un acide,
- c) on traite l'alcanolamine N-substituée ainsi obtenue de formule :

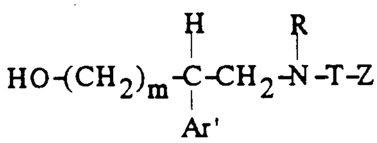 (V)

avec le chlorure de méthanesulfonyle ;

- d) on fait réagir le mésylate ainsi obtenu de formule :

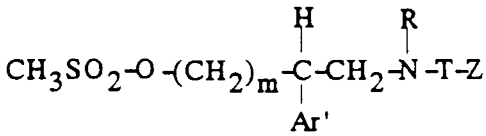 (VI)

avec une amine secondaire de formule :

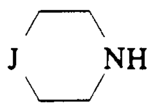 (VII)

dans laquelle J est tel que défini précédemment ; et

- e) après déprotection éventuelle de l'hydroxyle représenté par $X_1$, on transforme éventuellement le produit ainsi obtenu en un de ses sels pharmaceutiquement acceptables.

Pour préparer les nouveaux composés de formule (Ia) où J est un groupe

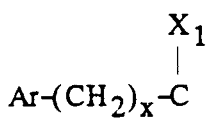

dans lequel Ar est tel que défini ci-dessus, x est zéro et $X_1$ est l'hydrogène, on fait réagir, dans l'étape (d), le composé (VI) avec une 4-Ar-pipéridine (Ar étant tel que défini ci-dessus).

Plus particulièrement, pour préparer les composés préférés de formule (Ia'), le mésylate de formule (VI) est mis en réaction avec la 4-phénylpipéridine (formule VII, J = benzylidène), bien connue en littérature.

Comme dérivé fonctionnel de l'acide (III), on utilise l'acide lui-même, convenablement activé par exemple par le cyclohexylcarbodiimide ou par l'hexafluorophosphonate de benzotriazolyl N-oxytrisdiméthylaminophosphonium (BOP), ou bien un des dérivés fonctionnels qui réagissent avec les amines, par exemple un anhydride, un anhydride mixte, le chlorure d'acide ou un ester activé.

Lorsque comme produit de départ on utilise un composé de formule (II) où E représente un groupe :

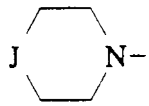

le procédé de préparation des composés (Ia) peut être représenté et illustré en détail par le SCHEMA 1 ci-après :

SCHEMA 1

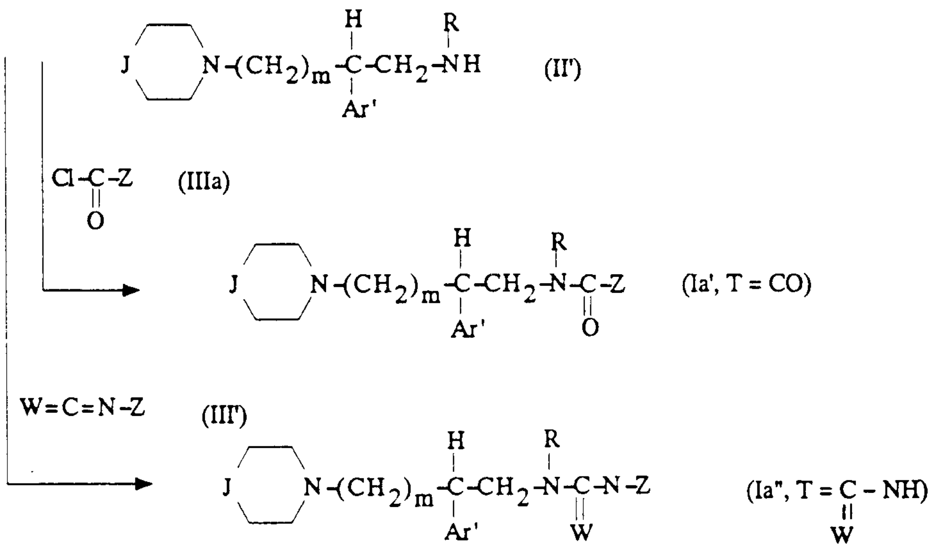

Dans la formule (IIIa) ci-dessus, on considère le chlorure d'acide comme dérivé fonctionnel réactif de l'acide (III). On peut cependant utiliser un autre dérivé fonctionnel ou on peut partir de l'acide libre (III) en réalisant un couplage de (II') avec le BOP, puis, en additionnant l'acide (III) en présence d'une base organique comme par exemple la triéthylamine, dans un solvant comme le dichlorométhane ou le diméthylformamide, à température ambiante ; les composés (Ia') obtenus sont isolés et purifiés selon les méthodes habituelles, comme par exemple la chromatographie ou la recristallisation.

On peut faire aussi réagir (II') avec un iso(thio)cyanate W = C = N-Z (III') dans un solvant inerte anhydre tel que par exemple le benzène, pendant une nuit à température ambiante puis traiter le mélange réactionnel selon les méthodes habituelles pour obtenir les composés (Ia").

Lorsque comme produit de départ on utilise un composé de formule (II) où E représente un groupe 2-tétrahydropyranyloxy, le procédé de la présente invention peut être représenté et illustré à partir du SCHEMA 2.

Les réactions du composé (II") avec les réactifs (IIIa) et (III') se déroulent comme décrit ci-dessus pour le Schéma 1, le chlorure d'acide (IIIa) pouvant être remplacé par un autre dérivé fonctionnel ou par l'acide libre activé par exemple par le BOP.

L'intermédiaire (IV') ainsi obtenu est déprotégé par hydrolyse acide douce pour conduire au composé hydroxylé libre (V) duquel on prépare le mésylate (VI) pour le substituer par une amine secondaire de formule (VII) pour obtenir finalement les composés (Ia).

SCHEMA 2

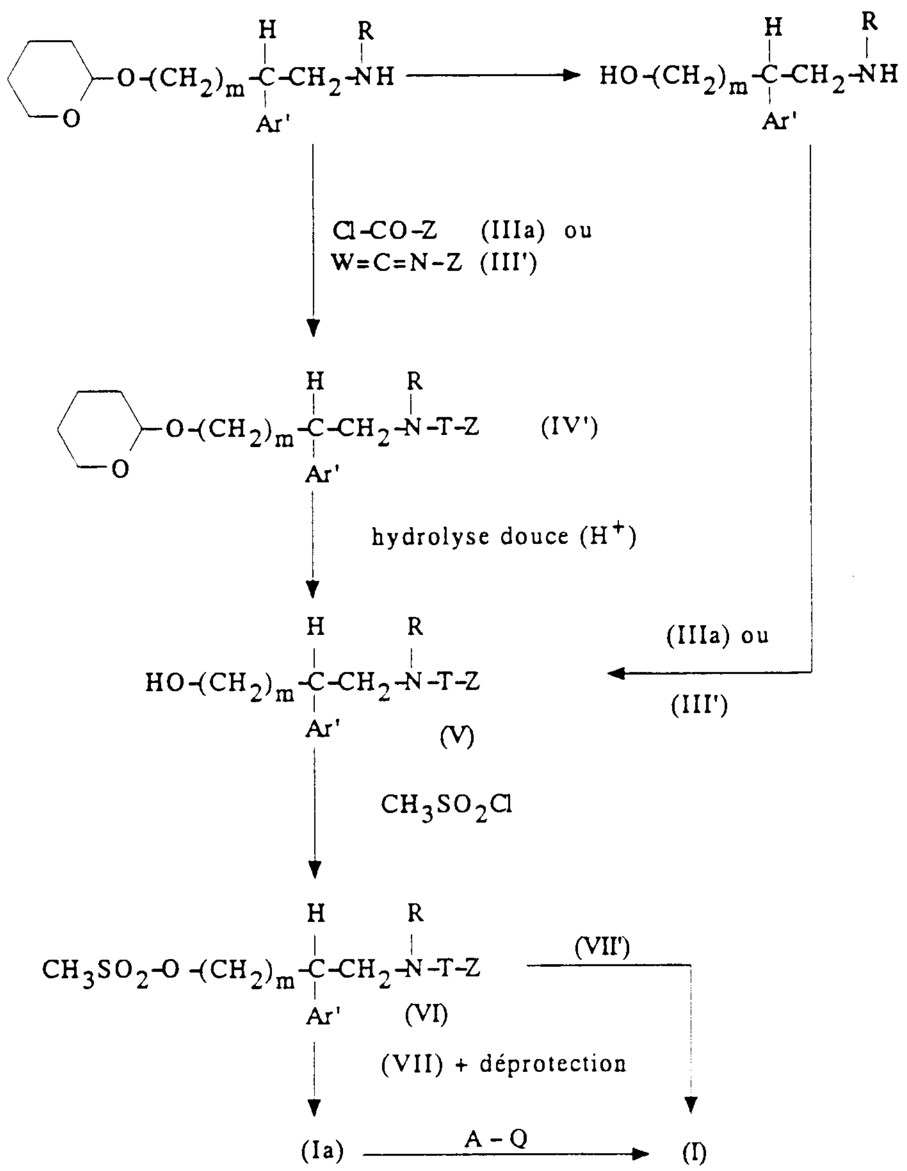


Les produits de formule (Ia) ainsi obtenus sont isolés, sous forme de base libre ou de sel, selon les techniques classiques.

Lorsque le composé de formule (Ia) est obtenu sous forme de base libre, il peut être purifié ou cristallisé par salification par traitement avec un acide dans un solvant organique. Ainsi, par traitement de la base libre, dissoute par exemple dans un alcool tel que l'isopropanol, avec une solution de l'acide choisi dans le même solvant, on obtient le sel correspondant qui est isolé selon les techniques classiques. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le 2-naphtalènesulfonate.

A la fin de la réaction, les composés de formule (Ia) ou, plus particulièrement, de formule (Ia') peuvent être isolés sous forme d'un de leurs sels, par exemple le chlorhydrate ou l'oxalate. La base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

La résolution des mélanges racémiques (Ia) ou, plus particulièrement, (Ia') permet d'isoler les énantiomères.

On peut aussi effectuer le dédoublement de mélanges racémiques des produits de formule (II), notamment des produits de formule (II") et (II"') ou de leurs précurseurs, afin de préparer les énantiomères des produits de formule (Ia) ou, plus particulièrement, de formule (Ia').

Les composés de départ de formule (II) sont préparés à partir de nitriles de formule :

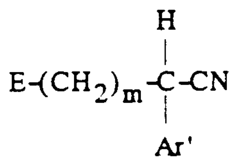

(VIII)

dans laquelle m, E et Ar' sont tels que définis ci-dessus, par réduction du nitrile.

Pour la préparation des composés de formule (II) où R est l'hydrogène, les nitriles de départ de formule (VIII) sont soumis à une hydrogénation dans un alcanol, tel que l'éthanol, en présence d'un catalyseur tel que le nickel de Raney, et l'amine libre primaire peut être isolée selon les méthodes classiques.

Lorsqu'on souhaite préparer les composés de formule (II) où R est le groupe méthyle, on traite l'amine libre, obtenue par hydrogénation du nitrile (VIII) comme décrit ci-dessus, avec un chloroformiate, par exemple avec le chloroformiate de formule Cl-CO-$OR_1$, où $R_1$ est un alkyle en $C_1$-$C_6$, pour obtenir les carbamates de formule :

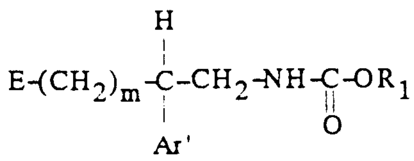

qui sont ensuite réduits par des moyens connus, tels que l'action d'un agent réducteur comme par exemple un hydrure métallique, tel que l'hydrure de sodium et d'aluminium, l'hydrure de lithium et d'aluminium ou par un hydrure de bore, tel que le diméthylsulfure de borane. La réduction est réalisée dans un solvant, tel que l'éther, le toluène ou le tétrahydrofurane, à une température comprise entre la température ambiante et 60°C. L'amine ainsi obtenue de formule :

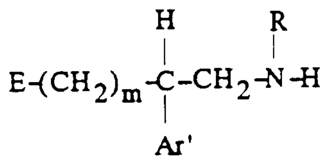

(II, R = $CH_3$)

est isolée selon les méthodes habituelles.

On peut aussi traiter le composé de formule :

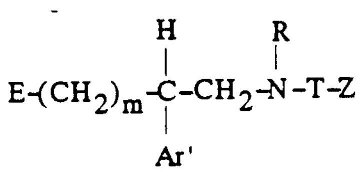

(IV, R = H)

dans laquelle m, E, Ar', T et Z sont tels que définis précédemment par un halogénure d'alkyle en présence d'une base forte telle que par exemple un hydrure métallique comme par exemple l'hydrure de sodium, dans un solvant inerte tel que le tétrahydrofurane chauffé au reflux pour préparer les composés (IV) dans lesquels R est autre que l'hydrogène.

Les nitriles de formule (VIII) sont préparés à partir de nitriles de formule :

$$Ar'\text{-}CH_2\text{-}CN \qquad (XI)$$

qui par alkylation avec un composé de formule :

$$E\text{-}(CH_2)_m\text{-}G \qquad (XII)$$

dans laquelle m et E sont tels que définis ci-dessus et G est un atome d'halogène, par exemple un atome de brome ou un groupe hydroxy protégé, donnent les composés (VIII) désirés.

La synthèse des nitriles de formule (VIII) où E est un groupe 2-tétrahydropyranyloxy est réalisée à partir d'un dérivé 2-tétrahydropyranyloxy (THP-O) obtenu par réaction entre un alcanol de formule $Br\text{-}(CH_2)_m\text{-}OH$ avec m tel que défini précédemment et le dihydropyrane pour conduire au compose :

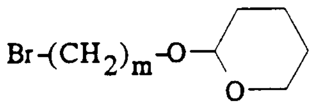

(XII, E = THP-O-, G = Br)

qui est ensuite additionné, en présence d'hydrure alcalin sur le dérivé acétonitrile (XI) pour préparer l'intermédiaire de formule :

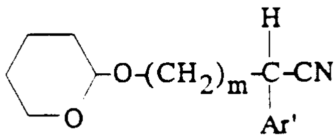

(VIII, E = THP-O-)

La synthèse des nitriles de formule (VIII) où E représente un groupe :

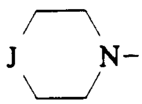

où J est tel que défini précédemment, est effectuée selon des méthodes connues par addition sur des dérivés chlorés de formule :

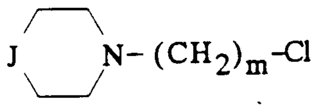

(XIII)

d'un dérivé nitrile de formule :

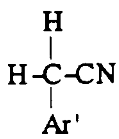

(XIV)

en présence d'amidure de sodium dans un solvant tel que le toluène à des températures comprises entre 30° et 80°C.

Le dérivé chloré (XIII) est préparé par action d'un réactif chlorant tel que le chlorure de thionyle sur le dérivé hydroxylé de formule :

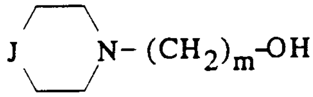

(XV)

lui-même préparé à partir de l'amine de formule (VII) dans laquelle, si $X_1$ = OH, le groupe hydroxyle est éventuellement protégé par un groupe O-protecteur selon les méthodes habituelles,

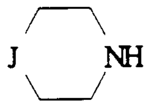

(VII)

amine sur laquelle on fait réagir, si m = 2, l'oxyde d'éthylène et, si m = 3, un halogéno-3 propanol.

Une alternative à la préparation des composés (I) selon l'invention consiste à faire réagir une amine tertiaire de formule :

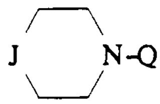

(VII')

dans laquelle J et Q sont tels que définis précédemment pour (I) avec le composé méthanesulfonate (VI) pour obtenir directement l'ammonium quaternaire (I) pour lequel $A^{\ominus}$ est représenté par l'anion méthanesulfonate. Cette réaction est réalisée selon les méthodes habituelles bien connues de l'homme de l'art. L'anion méthanesulfonate ainsi obtenu est ensuite éventuellement échangé par un autre anion choisi parmi $A^{\ominus}$ tel que défini ci-dessus, par exemple par l'anion chlorure en utilisant une résine échangeuse d'ions comme indiqué ci-dessus.

Les amines de formule (VII) et (VII') sont connues en littérature. Une méthode pour leur préparation est décrite dans le brevet GB 1060160.

Les composés selon l'invention ont fait l'objet d'essais biochimiques et pharmacologiques.

Ainsi, les composés selon l'invention antagonisent la liaison de la Substance P à son récepteur avec un Ki compris entre 0,1 et 800 nM dans des essais réalisés sur le cortex de rat et avec un Ki compris entre 0,01 et 30 nM dans des essais réalisés sur la lignée cellulaire lymphoblastique IM9.

Les composés de la présente invention sont peu toxiques ; notamment, leur toxicité aigüe est compatible avec leur utilisation comme médicament. Pour une telle utilisation, on administre aux mammifères une quantité efficace d'un composé de formule (I).

Les composés de la présente invention sont généralement administrés en unités de dosage. Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I).

Les composés de formule (I) ci-dessus peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Dans les compositions pharmaceutiques de la présente invention pour l'administration orale, sublinguale, sous-cutanée, intramusculaire, intraveineuse, transdermique, topique ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange le principe actif avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible.

Le principe actif peut être formulé également sous forme de microcapsules, éventuellement avec un ou plusieurs supports ou additifs.

Le principe actif peut être également présenté sous forme de complexe avec une cyclodextrine, par exemple α-, β-, γ-cyclodextrine, 2-hydroxypropyl-β-cyclodextrine, méthyl-β-cyclodextrine.

Les compositions susdites peuvent également renfermer d'autres produits actifs tels que, par exemple, des bronchodilatateurs, des antitussifs ou des antihistaminiques.

Dans chaque unité de dosage le principe actif de formule (I) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,5 à 1000 mg de principe actif, de préférence de 2,5 à 250 mg devant être administrés une à quatre fois par jour.

Selon une autre de ses aspects, la présente invention concerne l'utilisation des produits de formule (I) pour la préparation de médicaments destinés à traiter des troubles physiologiques associés à un excès de tachykinines, notamment de Substance P. Plus particulièrement, par exemple, les troubles du système nerveux central, les maladies neurodégénératives, les troubles respiratoires, les maladies inflammatoires, les troubles du système gastrointestinal, les troubles circulatoires, la douleur, la migraine. La présente invention inclut aussi une méthode pour traiter lesdites affections aux doses indiquées ci-dessus.

Les composés intermédiaires de formule (Ia) sont préparés selon le procédé décrit dans la présente description et selon les demandes de brevet EP-A-428434 et EP-A-0474561. La protection des intermédiaires de synthèse comportant des fonctions aminées est réalisée selon des méthodes connues, par exemple selon EP-A-512901.

Les composés (Ia) optiquement purs sont également obtenus selon le procédé décrit dans les demandes EP-A-428434 et EP-A-0474561.

Les composés de formule (Ia) dans laquelle J est

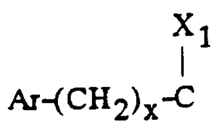

où Ar est tel que défini ci-dessus, x est zéro et $X_1$ est l'hydrogène, notamment les composés de formule (Ia'), sont eux aussi des puissants antagonistes de la Substance P, plus particulièrement, le composé (I'b), dont le Ki est compris entre 0,3 et 0,6 nM dans des essais réalisés respectivement sur le cortex de rat et sur la lignée cellulaire lymphoblastique IM9.

Les spectres de résonnance magnétique nucléaire du proton $^1H$ ont été effectués à 200 MHz. Les spectres de résonnance magnétique nucléaire du $^{13}C$ ont été effectués à 50 MHz. Les déplacements chimiques sont exprimés en ppm.

Les points de fusion, F, ont été mesurés au banc chauffant Koffler.

(a) ou (e) signifient la position axiale ou équatoriale du substituant Q.

Les exemples suivants illustrent l'invention sans toutefois la limiter.

**PREPARATIONS**

A. <u>AMINOALCOOLS</u>.

PREPARATION I.

(a) α-(2-Tétrahydropyranyloxyéthyl)-2-(3,4-dichlorobenzène)acétonitrile.

16,5 g d'hydrure de sodium à 80 % dans l'huile sont mis en suspension dans 200 ml de tétrahydrofurane sec. On ajoute goutte à goutte à 20°C, en 30 minutes, une solution de 100 g de 3,4-dichlorophénylacétonitrile dans 500 ml de tétrahydrofurane puis on agite le mélange réactionnel à température ambiante pendant 2 heures. Le mélange est refroidi à -20°C et on ajoute une solution de 118 g de 1-bromo-2-tétrahydropyranyl-oxyéthane dans 100 ml de tétrahydrofurane, on laisse revenir le mélange à température ambiante et après 2 heures on ajoute une solution de 50 g de chlorure d'ammonium dans 3 litres d'eau. On extrait avec 1,5 litres d'éther, lave avec une solution saturée de NaCl, décante, sèche sur $MgSO_4$, et concentre sous vide. Le résidu est chromatographié sur gel de silice, éluant : $CH_2Cl_2$ puis acétate d'éthyle 95-5 (v/v). Les fractions de produit pur sont concentrées sous vide pour fournir 118 g d'une huile.

(b) 2-(2-Tétrahydropyranyloxyéthyl)-2-(3,4-dichlorobenzène)éthanamine.

118 g du nitrile obtenu précédemment sont mis en solution dans 700 ml d'éthanol absolu. On ajoute 300 ml d'ammoniaque concentrée puis, sous balayage d'azote, on ajoute du nickel de Raney (10 % de la quantité de nitrile de départ). On hydrogène ensuite sous atmosphère d'hydrogène à température ambiante et pression ordinaire.
16 litres sont absorbés en 4 heures. Le catalyseur est séparé par filtration sur célite, le filtrat est concentré sous vide, le résidu est repris dans une solution saturée de NaCl. Après extraction à l'éther et séchage sur $MgSO_4$, on obtient 112 g d'une huile.

(c) 2-(2-Hydroxyéthyl)-2-(3,4-dichlorobenzène)éthanamine.

81 g du produit obtenu précédemment selon (b) sont dissous dans 38 ml de méthanol. On ajoute 80 ml d'une solution d'éther éthylique saturée d'acide chlorhydrique en maintenant la température entre 20 et 25°C. On agite pendant 30 minutes à température ambiante, puis concentre à sec. On dissout le résidu dans 250 ml d'eau, lave deux fois à l'éther éthylique, alcalinise avec une solution de NaOH, extrait au $CH_2Cl_2$. Après séchage sur $MgSO_4$, on concentre sous vide, à sec, reprend dans 800 ml d'éther isopropylique, sépare un insoluble par filtration sur Célite, concentre sous vide à environ 300 ml, amorce avec des cristaux d'aminoalcool, agite pendant une nuit. On filtre, rince à l'éther isopropylique puis au n-pentane. On obtient 30,2 g du produit attendu. F = 90-91°C.

(d) 2-(2-Hydroxyéthyl)-2-(3,4-dichlorobenzène)éthanamine (+).

A une solution bouillante de 29 g d'acide D (-) tartrique dans 800 ml de méthanol, on ajoute une solution de 44,7 g de produit obtenu selon l'étape (c) précédente dans 300 ml de méthanol. On laisse revenir à température ambiante et agite pendant 4 heures. On filtre, rince à l'éthanol puis à l'éther. On obtient 34,1 g de tartrate. On recristallise dans 1,75 l de méthanol pour obtenir 26,6 g de tartrate.

$[\alpha]_D^{25}$ = -4,2° (c = 1, dans $H_2O$)

Ce tartrate est repris dans 120 ml d'eau. On alcalinise avec une solution de NaOH, extrait deux fois au $CH_2Cl_2$, sèche sur $MgSO_4$, concentre sous vide, à sec. On reprend dans un peu d'éther isopropylique, ajoute du n-pentane, filtre pour obtenir 15,4 g du produit attendu. F = 79-80°C.

$[\alpha]_D^{25}$ = + 9,4° (c = 1, dans $CH_3OH$)

(e) Chlorhydrate de N-méthyl-2-(2-hydroxyéthyl)-2-(3,4-dichlorobenzène)éthanamine (+).

(e1) N-[4-Hydroxy-2-(3,4-dichlorophényl)butyl]carbamate d'ethyle.

15 g de produit obtenu selon l'étape (d) précédente sont dissous dans 200 ml de $CH_2Cl_2$. On ajoute 9,9 ml de triéthylamine. On refroidit à 0°C et ajoute goutte à goutte à cette température une solution de 6,3 ml de chloroformiate d'éthyle dans 30 ml de $CH_2Cl_2$. Après 15 minutes, on lave à l'eau puis avec une solution d'HCl diluée, puis avec une solution aqueuse saturée de $NaHCO_3$. Après séchage sur $MgSO_4$ on concentre sous vide, à sec, pour obtenir 20 g de produit sous forme d'huile.

(e2) Réduction du groupe éthoxycarbonyle en groupe méthyle.

A 5,1 g d'hydrure de lithium/aluminium en suspension dans 60 ml de THF anhydre, on ajoute une solution de 20 g de produit obtenu selon l'étape (e1) précédente dans 150 ml de THF anhydre. On chauffe à reflux pendant 1 heure. On hydrolyse avec 20 ml d'eau, sépare le minéral par filtration, concentre sous vide, à sec. L'huile obtenue est dissoute dans 100 ml d'acétone. On ajoute de l'éther éthylique saturé d'acide chlorhydrique jusqu'à pH = 1, puis de l'éther éthylique jusqu'au trouble. On agite pendant 1 heure, on filtre les cristaux, rince avec un peu d'acétone, puis avec de l'éther éthylique pour obtenir 11 g de chlorhydrate de N-méthyl-2-(2-hydroxyéthyl)-2-(3,4-dichlorobenzène)éthanamine. F = 129°C.

$[\alpha]_D^{25}$ = + 8,4° (c = 1, dans $CH_3OH$)

(f) Chlorhydrate de N-méthyl-2-(2-hydroxyéthyl)-2-(3,4-dichlorobenzène)éthanamine (-).

En procédant comme précédemment à partir de l'acide L (+) tartrique on obtient, l'énantiomère (-). F = 129°C

$[\alpha]_D^{20}$ = - 8,4° (c = 1, dans $CH_3OH$)

B. <u>ACIDES PHENYLACETIQUES</u>.

PREPARATION II.

1. ACIDE 3-ISOPROPOXYPHENYLACETIQUE.

L'acide 3-isopropoxyphénylacétique n'est pas connu dans la littérature mais peut être préparé selon des méthodes bien connues pour la préparation d'acides alcoxyphénylacétiques.

(a) 3-Hydroxyphénylacétate d'éthyle.

55 g d'acide 3-hydroxyphénylacétique en solution dans 400 ml d'éthanol 100 sont chauffés à reflux pendant une nuit en présence de quelques gouttes d'$H_2SO_4$ concentré. On évapore sous vide, à sec, reprend à l'éther éthylique, lave à l'eau puis avec une solution aqueuse saturée de $NaHCO_3$. Après séchage sur $MgSO_4$, puis évaporation on obtient 58 g d'une huile.

(b) 3-Isopropoxyphénylacétate d'éthyle.

58 g du produit obtenu précédemment, 88 g de $K_2CO_3$ et 108 g de 2-iodopropane, en solution dans 300 ml de DMF, sont chauffés à 80-100°C pendant 8 heures. On évapore le DMF sous vide, reprend à l'acétate d'éthyle et lave avec une solution aqueuse à 10 % de $K_2CO_3$. Après séchage sur $MgSO_4$ puis évaporation, on purifie le résidu par chromatographie sur gel de silice, éluant : $CH_2Cl_2$. On obtient ainsi 61 g d'une huile.

(c) Acide 3-isopropoxyphénylacétique.

31 g de produit obtenu précédemment et 20 g de NaOH en solution dans 400 ml d'éthanol, sont chauffés à reflux pendant 2 heures. On évapore à sec, reprend à l'eau et acidifie avec de l'HCl concentré. On extrait à l'éther éthylique lave à l'eau, sèche sur $MgSO_4$ et concentre sous vide, à sec, pour obtenir 27 g de l'acide attendu. F = 33-35°C.

De la même façon, on prépare l'acide 3-éthoxyphénylacétique.

PREPARATION II.

2. ACIDE (3-ISOPROPOXYPHENYL)HYDROXYACETIQUE.

(a) 3-Isopropoxybenzaldéhyde.

50 g de 3-hydroxybenzaldéhyde sont mis en solution, sous azote, dans 250 ml de DMF. On ajoute 60 g de $K_2CO_3$ puis 60 ml de 2-iodopropane, chauffe le mélange réactionnel pendant 18 heures à 50°C et le verse dans 2,5 litres d'eau. On extrait à l'éther éthylique, lave avec une solution de NaOH diluée, sèche la phase éthérée sur $MgSO_4$ et concentre sous vide pour obtenir 53,5 g d'huile.

(b) (3-Isopropoxyphényl)hydroxyacétonitrile.

A 1 g du produit préparé précédemment en suspension dans 3 ml d'eau on ajoute 0,72 g de bisulfite de sodium. Le mélange est laissé pendant 18 heures à température ambiante puis on ajoute une solution de 0,85 g de KCN dans 2 ml d'eau et agite le mélange réactionnel pendant 30 minutes. On extrait à l'éther éthylique, lave à l'eau, sèche sur $Na_2SO_4$ et concentre sous vide pour obtenir 1,2 g du composé attendu sous forme d'huile.

(c) Acide (3-isopropoxyphényl)hydroxyacétique.

Un mélange de 1 g du produit obtenu précédemment dans 1,1 ml d'eau et 1,1 ml d'HCl concentré est chauffé à reflux pendant une heure. On laisse refroidir la solution, extrait à l'éther éthylique, lave la phase éthérée à l'eau et extrait avec une solution de NaOH diluée. La phase aqueuse est acidifiée avec HCl puis on extrait à l'éther éthylique, sèche sur $MgSO_4$ et concentre sous vide. L'acide est cristallisé dans un mélange d'éther isopropylique/pentane (50/50) pour fournir 0,42 g du produit attendu ; F = 94°C.

C. <u>DERIVES ACYLES ET DERIVES SULFONYLOXY.</u>

PREPARATION III.

1. Acylation via l'acide.

2-(2-Hydroxyéthyl)-2-(3,4-dichlorophényl)-N-(3-isopropoxyphényl)-N-méthyl-acétamide.

A 2,8 g d'acide 3-isopropoxyphénylacétique en solution dans 50 ml de $CH_2Cl_2$ on ajoute 6,05 ml de triéthylamine puis 5,05 g de N-méthyl-2-(2-hydroxyéthyl)-2-(3,4-dichlorobenzène)éthanamine préparée selon EP-A-474561 et 7,08 g de BOP. Après 4 heures d'agitation à température ambiante, le mélange est concentré sous vide, le résidu est repris dans l'acétate d'éthyle et on lave successivement à l'eau, avec une solution de NaOH diluée, avec une solution saturée de NaCl. La phase organique est séchée sur $MgSO_4$, filtrée et concentrée sous vide et le résidu est chromatographié sur gel de silice, éluant : $CH_2Cl_2$-AcOEt, 96-4 (v/v). La concentration des fractions de produit pur fournit 7,0 g du composé acylé attendu.

2. Acylation via le chlorure d'acide.

2-(2-Hydroxyéthyl)-2-(3,4-dichlorophényl)-N-méthyl-2-thiophènecarboxamide (-).

A 8,1 g de produit obtenu selon la PREPARATION I (e) précédente, en suspension dans 120 ml de dichlorométhane, on ajoute 8,4 ml de triéthylamine. On refroidit à 0°C et on ajoute goutte à goutte une solution de 2,97 g de chlorure de l'acide 2-thiophènecarboxylique dans 35 ml de dichlorométhane. Après 15 minutes, on lave à l'eau, avec une solution d'HCl dilué, puis une solution aqueuse de $NaHCO_3$. On sèche sur $MgSO_4$, filtre et concentre sous vide, à sec. On obtient 9,0 g d'un solide qui est repris dans l'éther éthylique et filtré. F = 107-108°C.

$[\alpha]_D^{20}$ = -47,2° (c = 1, $CH_3OH$).

PREPARATION IV.

1. N-[4-méthanesulfonyloxy-2-(3,4-dichlorophényl)butyl]-N-méthyl-3-isopropoxy-phénylacétamide.

11,7 g du produit préparé précédemment selon la PREPARATION III 1. sont mis en solution dans 100 ml de $CH_2Cl_2$ en présence de 6,68 g de triéthylamine, puis on ajoute goutte à goutte 6,94 g de chlorure de méthanesulfonyle. Le mélange réactionnel est laissé pendant une nuit à température ambiante puis concentré sous vide. Le résidu est repris dans l'acétate d'éthyle et on lave successivement à l'eau puis avec une solution saturée de NaCl, décante la phase organique, sèche sur $MgSO_4$, filtre et concentre sous vide. L'huile résiduelle est concentrée sur gel de silice, éluant : $CH_2Cl_2$-$CH_3OH$, 99-3, (v/v). La concentration des fractions de produit pur fournit 11,04 g du mésylate attendu.

2. N-méthyl-N-[4-méthanesulfonyloxy-2-(3,4-dichlorophényl)butyl]-2-thiophène-carboxamide (-).

A 10,71 g de produit obtenu selon la PREPARATION III 2. précédente, en solution dans 120 ml de $CH_2Cl_2$, on ajoute 4,8 ml de triéthylamine. On refroidit à 0°C et on ajoute goutte à goutte 2,7 ml de chlorure de méthanesulfonyle. Après 15 minutes, on lave 2 fois à l'eau puis avec une solution aqueuse saturée de NaCl. On sèche sur $MgSO_4$, filtre et concentre sous vide, à sec pour obtenir une mousse.

D. <u>DERIVES AMINES</u>

PREPARATION V.

1. Chlorhydrate de N-[4-(4-phényl-1-pipéridinyl)-2-(3,4-dichlorophényl)butyl]-N-méthyl-3-isopropoxyphénylacétamide.

2 g du composé obtenu selon la PREPARATION IV 1. et 1,41 g de 4-phénylpipéridine sont mis en solution dans 10 ml de DMF et le mélange réactionnel est chauffé à 70°C pendant 3 heures. On concentre la solution sous vide, reprend le résidu dans l'AcOEt, et lave successivement à l'eau puis avec une solution saturée de NaCl, décante la phase organique, sèche sur $MgSO_4$, filtre et concentre sous vide. Le résidu est chromatographié sur gel de silice, éluant : $CH_2Cl_2$-$CH_3OH$, 97-3 (v/v). Les fractions de produit pur sont concentrées sous vide puis la base ainsi obtenue sous forme d'huile est dissoute dans l'éther éthylique et on fait barbotter de l'acide chlorhydrique gazeux pour en préparer le chlorhydrate. On obtient 0,27 g du produit attendu. F = 89-91°C.

2. N-[4-(4-hydroxy-4-phényl-1-pipéridinyl)-2-(3,4-dichlorophényl)butyl]-N-méthyl-2-thiophènecarboxamide (-).

12,12 g du produit obtenu selon la PREPARATION IV 2. précédente et 11,8 g de 4-hydroxy-4-phénylpipéridine sont mis en solution dans 30 ml de DMF et chauffés à 70°C pendant une heure et demie. La solution est versée dans 30 ml d'eau glacée, on sépare un précipité par filtration et on le sèche. Le précipité obtenu est purifié par chromatographie sur gel de silice, éluant : $CH_2Cl_2$-$CH_3OH$, 90-10 (v/v). Les fractions de produit pur sont concentrées sous vide puis, le résidu est cristallisé dans l'éther. F = 120-121°C.

On prépare le chlorhydrate dans l'acétone par addition d'une solution d'éther éthylique saturé d'acide chlorhydrique jusqu'à pH = 1. Le précipité est séparé par filtration et séché.

$[\alpha]_D^{20}$ = -51,0° (c = 1, $CH_3OH$).

3. Chlorhydrate de N-[4-(4-hydroxy-4-phényl-1-pipéridinyl)-2-(3,4-dichlorophényl)-butyl]-N-méthyl-4-fluoro-1-naphtylamide.

1,5 g de N-méthyl-N-[4-méthanesulfonyloxy-2-(3,4-dichlorophényl)butyl]-4-fluoro-1-naphtylamide et 1,28 g de 4-hydroxy-4-phénylpipéridine sont chauffés à 80-90°C dans 8 ml de DMF pendant 3 heures. On laisse revenir le mélange réactionnel à température ambiante puis on le verse dans l'eau, extrait à l'acétate d'éthyle et lave successivement la phase organique à l'eau et avec une solution saturée de NaCl. La phase organique est ensuite séchée sur $MgSO_4$, on filtre et concentre sous vide. L'huile résiduelle est chromatographiée sur gel de silice H, éluant : $CH_2Cl_2$-$CH_3OH$, 98-2, (v/v). Le chlorhydrate des fractions de produit pur est préparé puis recristallisé dans un mélange acétone et acétate d'éthyle pour fournir 2,1 g du produit attendu. F = 180°C.

4. Chlorhydrate de N-[4-(4-benzyl-1-pipéridinyl)-2-(3,4-dichlorophényl)butyl]-N-méthyl-3-éthoxy-1-phénylacétamide.

A une solution de 2 g de N-(4-benzyl-1-pipéridinyl)-2-(3,4-dichlorophényl)-N-méthylbutylamine et de 0,8 g d'acide 3-éthoxyphénylacétique dans 40 ml de $CH_2Cl_2$, on ajoute 2 ml de triéthylamine puis 2,2 g de BOP. Le mélange réactionnel est laissé pendant une heure à température ambiante puis concentré sous vide. Le résidu est repris dans l'acétate d'éthyle et on lave successivement à l'eau, avec une solution diluée de NaOH puis avec une solution saturée de NaCl. La phase organique est séchée sur $MgSO_4$, filtrée et concentrée sous vide. Le résidu est purifié par chromatographie sur gel de silice, éluant : $CH_2Cl_2$-$CH_3OH$, 100 + 1,7 (v/v). On prépare le chlorhydrate des fractions de produit pur et on obtient 1,65 g du chlorhydrate attendu. F = 130°C.

5. Chlorhydrate de N-[4-(4-benzyl-1-pipéridinyl)-2-(3,4-dichlorophényl)butyl]-N-méthyl-3,5-diméthoxyphénylacétamide.

En procédant comme précédemment selon la PREPARATION V 4. et en utilisant l'acide 3,5-diméthoxyphénylacétique préparé selon Tetrahedron Letters, 1991, *32*, 23, 2663-2666 on obtient le produit attendu. F = 150°C.

Les composés de formule (Ia) précurseurs non quaternaires des composés (I) selon l'invention, répertoriés dans le TABLEAU A ci-après, ont été préparés en mettant en oeuvre les PREPARATIONS indiquées ci-dessus.

## TABLEAU A

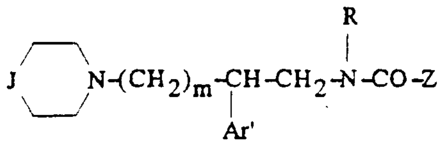

(Ia)

| J | m | Ar' | R | Z | F; °C |
|---|---|---|---|---|---|
| 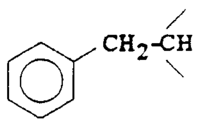 | 2 | 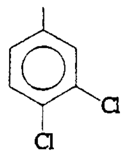 | $CH_3$ | 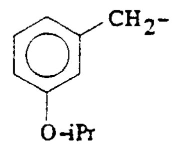 | 123 |
| 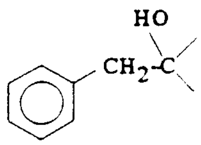 | 2 | 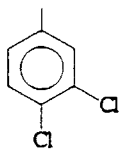 | $CH_3$ | 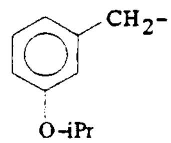 | 134 |
| 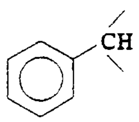 | 2 | 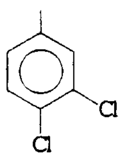 | $CH_3$ | 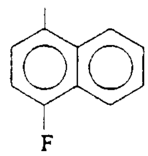 | énantiomère (-) 140 |
| 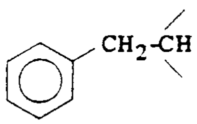 | 3 | 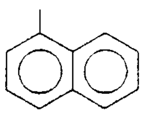 | H | 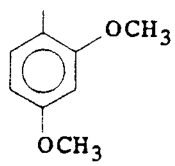 | 121 |

TABLEAU A (suite)

| | | | | | |
|---|---|---|---|---|---|
| 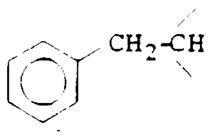 | 2 | 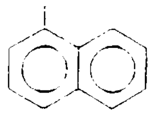 | H | 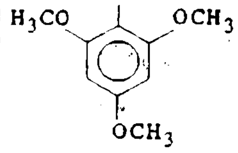 | 132 |
| 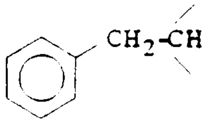 | 2 | 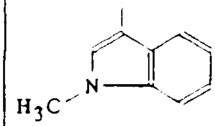 | H | 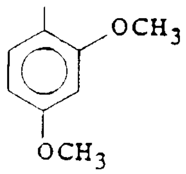 | 124 |

EXEMPLE 1 (Composé 1).

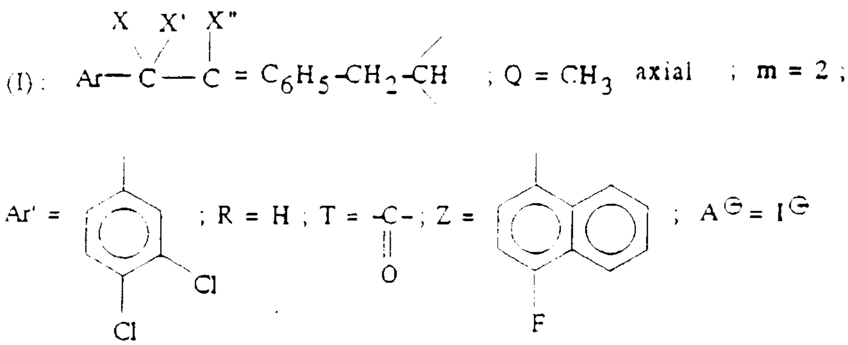

2 g de N-[4-(4-benzyl-1-pipéridinyl)-2-(3,4-dichlorophényl)butyl]-4-fluoro-1-naphtyl-amide préparés comme indiqué ci-dessus selon EP-A-428434 sont mis en solution dans 20 ml d'iodure de méthyle. La solution est agitée à température ambiante pendant 24 heures puis concentrée sous vide. Le résidu est chromatographié sur gel de silice, éluant: $CH_2Cl_2$-$CH_3OH$ 97/3 (v/v), puis $CH_2Cl_2$-$CH_3OH$ 95/5 (v/v). Le premier produit élué correspond à celui dont le méthyle en position 1 sur l'azote de la pipéridine est en position axiale. La fraction correspondante est concentrée sous vide pour fournir 1,3 g d'iodure de 1-[3-(3,4-dichlorophényl)-4-(4-fluoro-1-naphtoylamino)]butyl]-4-benzyl-N(a)-méthyl-1-pipéridinium. F = 120-122°C.

RMN $^1$**H** : 7 H entre 1,3 et 2,4 (2C**H**$_2$ et C**H** pipéridine, -C**H**$_2$β) ; 14 H entre 2,45 et 3,9 (2C**H**$_2$N pipéridine, C**H**$_2$ Ar, N $^+$ C**H**$_3$, N $^+$ C**H**$_2$, C**H** Ar', NH-C**H**$_2$) ; 14 H entre 7 et 8,2 (tous les **H** aromatiques) ; 1 H à 8,5 (N**H**) ; DMSO à 2,49.

EXEMPLE 2 (Composé 2).

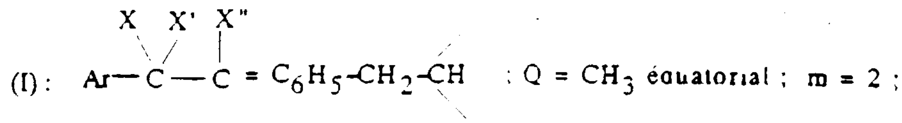

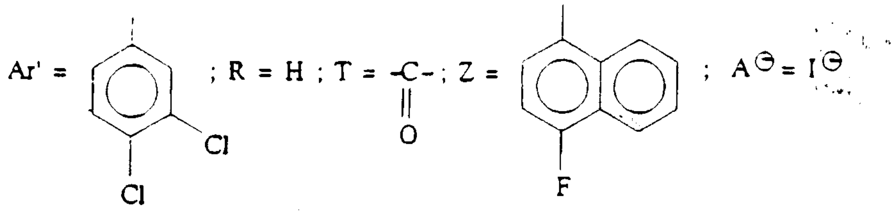

En procédant comme décrit précédemment à l'EXEMPLE 1 et en recueillant la fraction éluée en deuxième lieu, on obtient 0,3 g de l'iodure de 1-[3-(3,4-dichlorophényl)-4-(4-fluoro-1-naphtoylamino)]butyl]-4-benzyl-N(e)-méthyl-1-pipéridinium dont le méthyle en position 1 sur l'azote de la pipéridine est en position équatoriale. F = 120-122°C.

RMN : $^{1}$**H** : 7 H entre 1,3 et 2,4 (2C**H**$_2$ et C**H** pipéridine, -C**H**$_2\beta$); 14 H entre 2,45 et 4 (2C**H**$_2$N pipéridine, C**H**$_2$ Ar, N $^+$ CH$_3$, N $^+$ CH$_2$, C**H** Ar', CH$_2$ NH) ; 14 H entre 7 et 8.3 (tous les **H** aromatiques) ; 1 H à 8,6 (N**H**) ; DMSO à 2,49.

EXEMPLE 3 (Composé 3).

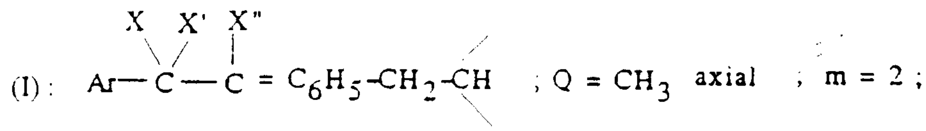

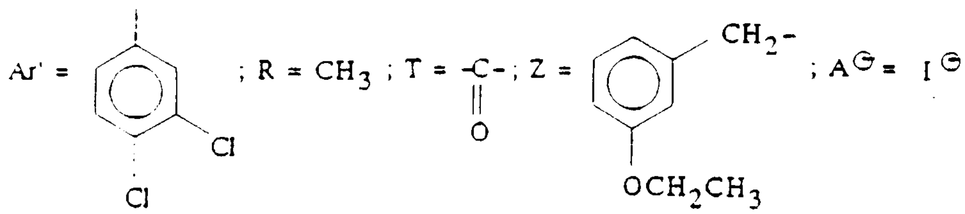

En procédant selon l'EXEMPLE 1 et en utilisant le N-[4-(4-benzyl-1-pipéridinyl)-2-(3,4-dichlorophényl)butyl]-N-méthyl-3-éthoxy-1-phénylacétamide comme produit de départ on prépare l'iodure de 1-[3-(3,4-dichlorophényl)-4-(N-méthyl-3-éthoxyphényl-1-acétamido)butyl]-4-benzyl-N(a)-méthyl-1-pipéridinium. F = 90°C.

EXEMPLE 4 (Composé 4).

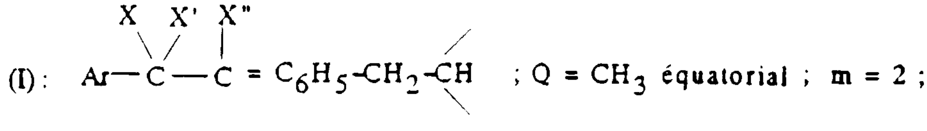

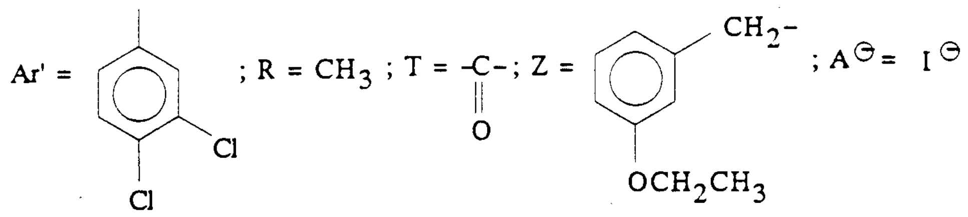

En procédant selon l'EXEMPLE 2 et en utilisant le N-[4-(4-benzyl-1-pipéridinyl)-3-(3,4-dichlorophényl)butyl]-N-méthyl-3-éthoxy-1-phénylacétamide comme produit de départ on prépare l'iodure de 1-[3-(3,4-dichlorophényl)-4-(N-méthyl-3-éthoxy-phényl-1-acétamido)butyl]-4-benzyl-N(e)-méthyl-1-pipéridinium. F = 105°C.

EXEMPLE 5 (Composé 5).

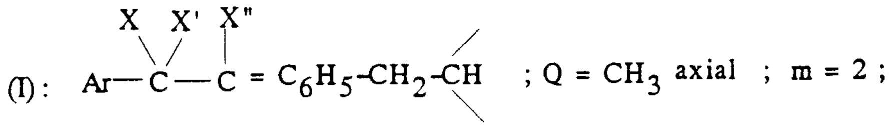

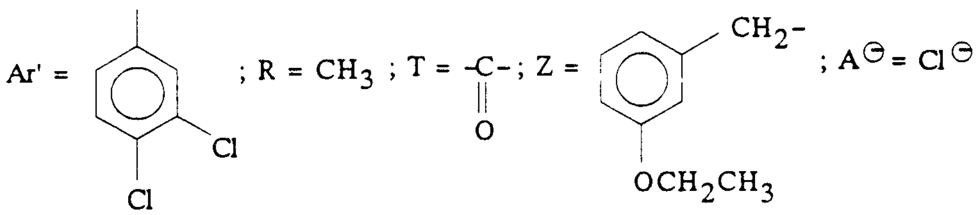

1 g de l'iodure obtenu selon l'EXEMPLE 3 (composé 3) est mis en solution dans 5 ml d'éthanol à 95°. On conditionne 40 ml de résine Duolite A 375 ® sous forme de chlorure et on élue sur cette colonne la solution préparée précédemment en rinçant ensuite à l'éthanol 95°. L'éluat est concentré sous vide et le résidu est repris dans l'éther. On filtre pour obtenir 0,69 g du chlorure de 1-[3-(3,4-dichlorophényl)-4-(N-méthyl-3-éthoxyphényl-1-acétamido)butyl]-4-benzyl-N(a)-méthyl-1-pipéridinium. F = 105°C.

EXEMPLE 6 (Composé 6).

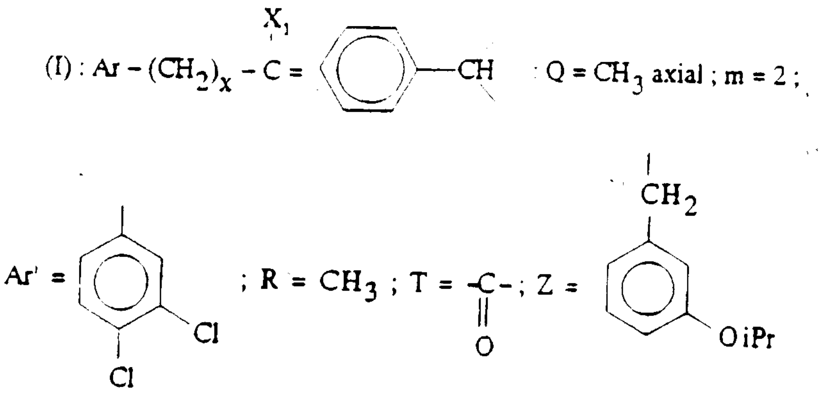

En procédant selon l'EXEMPLE 1 et en faisant réagir 1,1 g de N-[4-(4-phényl-1-pipéridinyl)-2-(3,4-dichlorophényl)butyl]N-méthyl-3-isopropoxyphénylacétamide préparé selon la PREPARATION V 1., avec 10 ml d'iodure de méthyle, on obtient en recueillant la première fraction éluée de la chromatographie, 0,53 g d'iodure de 1-[3-(3,4-dichlorophényl)-4-(N-méthyl-3-isopropoxyphénylacétylamino)butyl]-4-phényl-N(a)méthyl-1-pipéridinium. F = 105-107°C.

RMN $^{13}C$ : N $^{\oplus}$ - $CH_3$ : 33 ; N $^{\oplus}$ - $CH_2$- : 56.

EXEMPLE 7 (Composé 7).

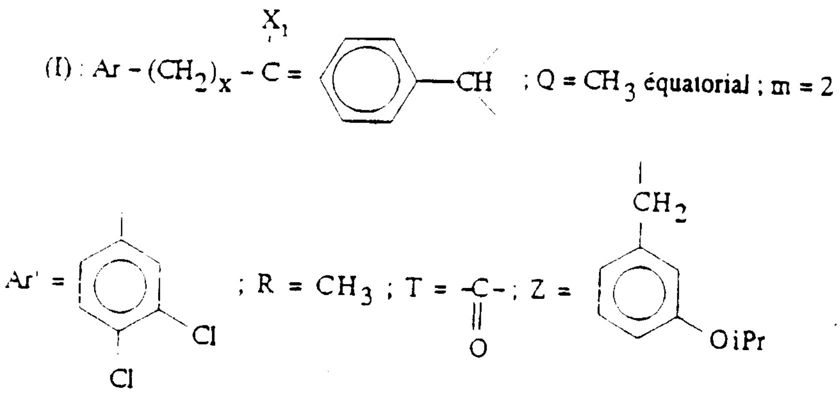

En procédant selon l'EXEMPLE 6 et en recueillant la deuxième fraction éluée de la chromatographie, on obtient l'iodure de 1-[3-(3,4-dichlorophényl)-4-(N-méthyl-3-isopropoxyphénylacétylamino)butyl]-4-phényl-N(e)-méthyl-1-piperidinium. F = 112-114°C.

RMN $^{13}C$ : N $^{\oplus}$ -$CH_3$ : 44 ; N $^{\oplus}$ -$CH_2$- : 46. EXEMPLE 8 (Composé 8)

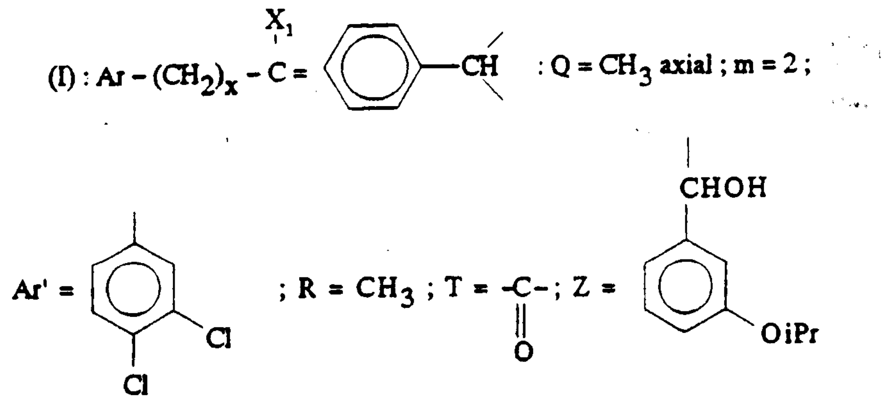

A) A 5,85 g de 2-(2-hydroxyéthyl)-2-(3,4-dichlorobenzène)éthaneamine obtenue selon la PREPARATION I (c) en solution dans 150 ml d'AcOEt on ajoute 6 g de dicarbonate de di(*tert*-butyle) dans 25 ml d'AcOEt, laisse le mélange à température ambiante pendant 20 minutes puis chauffe à reflux pendant 30 minutes. On concentre sous vide et on obtient 8,35 g de N-[4-hydroxy-2-(3,4-dichlorophényl)butyl]carbamate de *tert*-butyle.

B) A 8,35 g du carbamate obtenu précédemment en solution dans 100 ml de $CH_2Cl_2$ on ajoute à -20°C, 4 ml de triéthylamine et 2,14 ml de chlorure de mésyle. Le mélange réactionnel est agité à 0°C pendant 2 heures puis extrait à l'éther diéthylique. La phase éthérée est séchée sur $MgSO_4$, filtrée et concentrée sous vide pour fournir 9,87 g de N-[4-méthanesulfonyloxy-2-(3,4-dichlorophényl)butyl]carbamate de *tert*-butyle.

C) 9,87 g du mésylate préparé précédemment et 8,05 g de phényl-4-pipéridine sont mis en solution dans 20 ml de DMF et le mélange réactionnel est chauffé à 60°C pendant 2 heures. On ajoute 100 ml d'eau et extrait à l'AcOEt. La phase organique est décantée, puis successivement on sèche sur $Na_2SO_4$, filtre et concentre sous vide pour obtenir 11,8 g de N-[4-(4-phényl-1-pipéridinyl)-2-(3,4-dichlorophényl)butyl]carbamate de *tert*-butyle.

D) A 2,9 g d'hydrure de lithium aluminium en suspension dans 40 ml de THF anhydre, on ajoute une solution de 11,8 g du produit préparé précédemment en solution dans 30 ml de THF anhydre et chauffe le mélange réactionnel à reflux pendant 2 heures. On hydrolyse en additionnant 15 ml d'eau, sépare la minéral par filtration et concentre sous vide. On reprend le résidu dans 20 ml de $CH_2Cl_2$ et 10 ml d'une solution d'éther diéthylique saturé d'HCl puis concentre sous vide pour obtenir 6,7 g de N-[4-(4-phényl-1-pipéridinyl)-2-(3,4-dichlorophényl)butyl]-N-méthylamine qui cristallise dans l'acétone.

E) A 3 g de l'amine préparée précédemment en solution dans 60 ml de $CH_2Cl_2$ on additionne successivement 3,17 ml de triéthylamine, 1,40 g d'acide (3-isopropoxyphényl)hydroxyacétique obtenu selon la PREPARATION II. 2. et 3,14 g de BOP. Le mélange réactionnel est agité à température ambiante pendant une heure puis successivement lavé à l'eau puis avec une solution de $NaHCO_3$. On décante la phase organique, sèche sur $Na_2SO_4$, filtre et concentre sous vide. Le résidu est chromatographié sur gel de silice, éluant : $CH_2Cl_2$-$CH_3OH$, 97/3 (v/v). La concentration des fractions de produit pur fournit un résidu huileux auquel on additionne une solution d'éther diéthylique saturée d'HCl pour obtenir 2 g de dichlorhydrate de N-[4-(4-phényl-1-pipéridinyl)-2-(3,4-dichlorophényl)butyl]-N-méthyl-α-hydroxyphénylacétamide.

F) On fait réagir de l'iodure de méthyle sur l'acétamide préparé précédemment, dans les mêmes conditions que pour les exemples précédents et on obtient l'iodure de 1-[3-(3,4-dichlorophényl)-4-(N-méthyl-α-hydroxyphénylacétamido)butyl]-4-phényl-N(a)-méthyl-1-pipéridinium ; F = 130°C.

En procédant selon les PREPARATIONS précédentes et selon les EXEMPLES 1 à 8 ci-dessus, on prépare les composés 9 à 33 décrits dans les TABLEAUX I et II ci-après.

## TABLEAU I

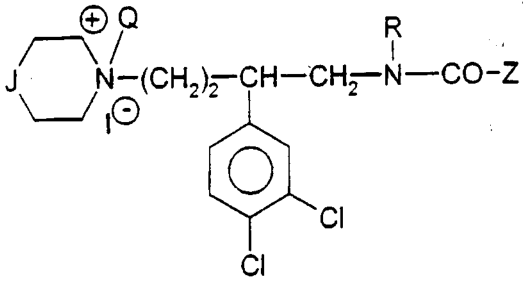

| Exemple n° | 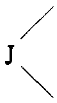 | Q (1) | C (2) | R | Z | F ; °C |
|---|---|---|---|---|---|---|
| 9 | 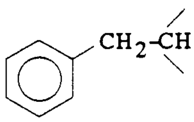 | $CH_3$ (a) | (+,–) | $CH_3$ | 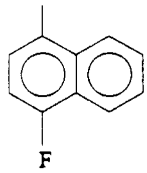 | 127 |
| 10 | 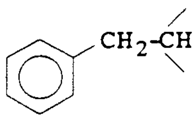 | $CH_3$ (a) | –25,6 | $CH_3$ | 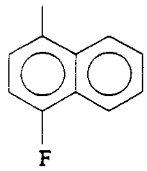 | 158–160 |
| 11 | 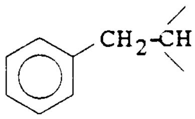 | $CH_3$ (a) | (+,–) | $CH_3$ | 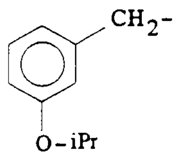 | 129–145 |
| 12 | 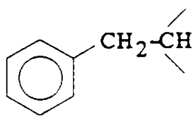 | $CH_3$ (e) | (+,–) | $CH_3$ | 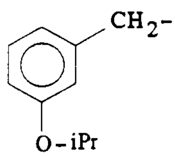 | 136–155 |

## TABLEAU I (suite 1)

| | | | | | | |
|---|---|---|---|---|---|---|
| 13 | 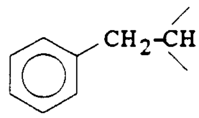 | $CH_3$ (a) | (+,–) | $CH_3$ | 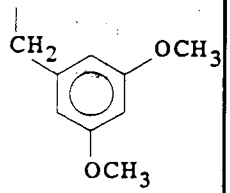 | 100 |
| 14 | 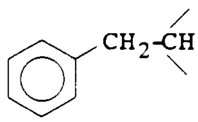 | $CH_3$ (e) | (+,–) | $CH_3$ | 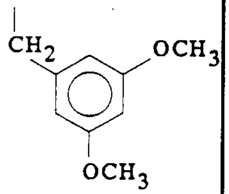 | 115 |
| 15 | 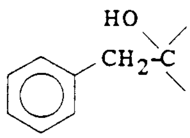 | $CH_3$ (a) | (+,–) | $CH_3$ | 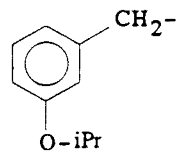 | 125–127 |
| 16 | 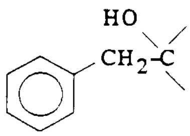 | $CH_3$ (e) | (+,–) | $CH_3$ | 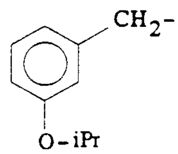 | 162–164 |
| 17 | 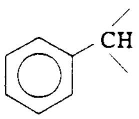 | $CH_3$ (a) | –35,5 | $CH_3$ | 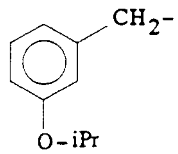 | 101–103 |

## TABLEAU I (suite 2)

| | | | | | | |
|---|---|---|---|---|---|---|
| 18 | 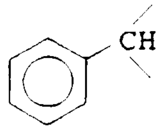 | $CH_3$ (e) | -7,6 | $CH_3$ | 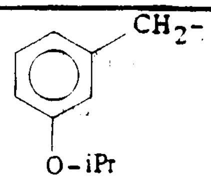 | 105-107 |
| 19 | 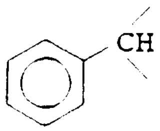 | $CH_3$ (a) | -31,9 | $CH_3$ | 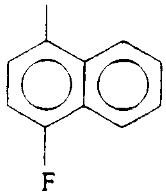 | 162-164 |
| 20 | 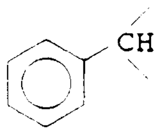 | $CH_3$ (e) | +1,9 | $CH_3$ | 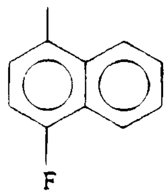 | 154-156 |
| 21 | 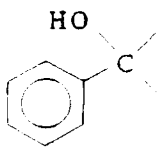 | $CH_3$ (a) | (+,-) | $CH_3$ | 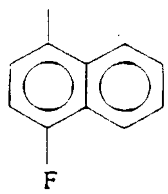 | 157 |

(1) : Configuration du substituant Q : (a) = axial, (e) = équatorial ; (2) : Configuration absolue du carbone asymétrique et $\alpha_D$ en ° à 25°C, C = 1 dans $CH_3OH$.

## TABLEAU II

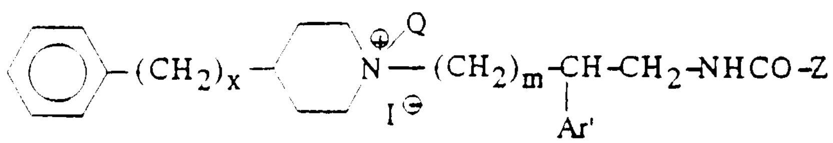

| Exemple n° | x | Q (1) | m | Ar' | Z | F; °C |
|---|---|---|---|---|---|---|
| 22 | 0 | $CH_3$ (a) | 2 | 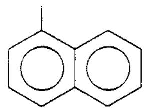 | 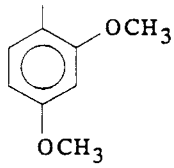 | 130-132 |
| 23 | 0 | $CH_3$ (e) | 2 | 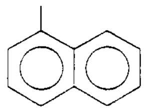 | 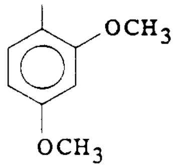 | 125-127 |
| 24 | 1 | $CH_3$ (e) | 3 | 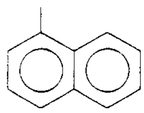 | 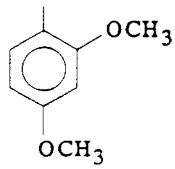 | 132 |
| 25 | 1 | $CH_3$ (a) | 3 | 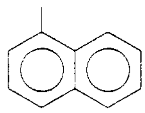 | 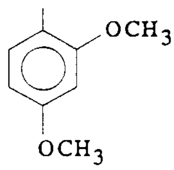 | 123 |
| 26 | 1 | $CH_3$ (a) | 2 | 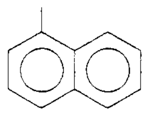 | 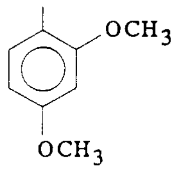 | 128-130 |

**TABLEAU II (suite)**

| | | | | | | |
|---|---|---|---|---|---|---|
| 27 | 1 | $CH_3$ (e) | 2 | 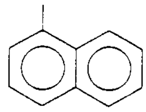 | 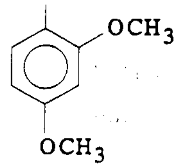 | 116-118 |
| 28 | 1 | $CH_3$ (a) | 2 | 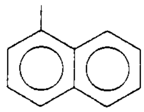 | 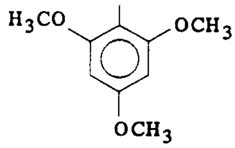 | 124 |
| 29 | 1 | $CH_3$ (e) | 2 | 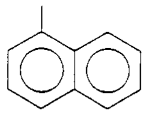 | 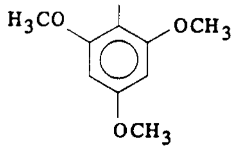 | 143 |
| 30 | 1 | $CH_3$ (a) | 2 | 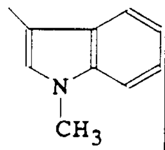 | 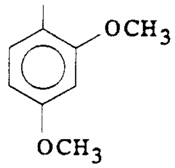 | 134 |
| 31 | 1 | $CH_3$ (e) | 2 | 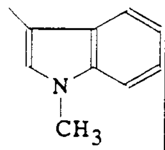 | 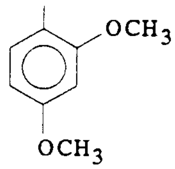 | 138 |
| 32 | 1 | $CH_3$ (a) | 2 | 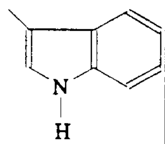 | 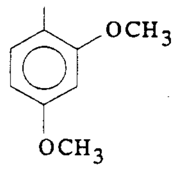 | 135-140 |
| 33 | 1 | $CH_3$ (e) | 2 | 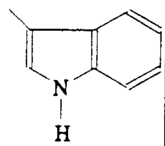 | 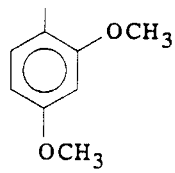 | 98 |

(1) : Configuration du substituant Q : (a) = axial, (e) = équatorial.

## Revendications

1. Sel d'ammonium quaternaire de formule :

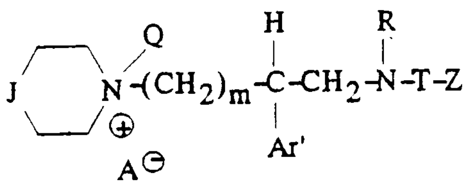

(I)

dans laquelle :

- J représente
  - * - soit un groupe

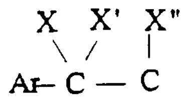

dans lequel

. Ar représente un phényle non substitué ou substitué une ou plusieurs fois par un substituant choisi parmi : l'hydrogène, un atome d'halogène, un hydroxyle, un alcoxy en $C_1$-$C_3$, un alkyle en $C_1$-$C_3$, un trifluorométhyle, lesdits substituants étant identiques ou différents ; un groupe cycloalkyle en $C_3$-$C_7$ ; un groupe pyridyle ou un groupe thiényle ;
. X représente l'hydrogène ;
. X' représente l'hydrogène ou est réuni à X'' ci-dessous pour former une liaison carbone-carbone ou bien X et X' ensemble forment un groupe oxo ;
. X'' est l'hydrogène ou avec X' forme une liaison carbone-carbone ;

  - * - soit un groupe

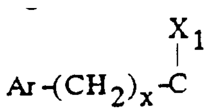

dans lequel :

. Ar est tel que défini ci-dessus ;
. x est zéro ou un ;
. $X_1$ représente l'hydrogène, uniquement lorsque x est zéro ; un hydroxyle; un alcoxy en $C_1$-$C_4$ ; un acyloxy en $C_1$-$C_4$ ; un carboxy ; un carbalcoxy en $C_1$-$C_4$ ; un cyano ; un groupe -NH-CO-Alk dans lequel Alk représente un alkyle en $C_1$-$C_6$ ; un groupe mercapto ; ou un groupe alkylthio en $C_1$-$C_4$ ; ou bien $X_1$ forme avec l'atome de carbone auquel il est lié et avec l'atome de carbone voisin dans la pipéridine une double liaison ;

- Q représente un groupe alkyle en $C_1$-$C_6$ ou un groupe benzyle ;
- $A^{\ominus}$ représente un anion choisi parmi chlorure, bromure, iodure, acétate, méthanesulfonate, paratoluènesulfonate ;
- m est 2 ou 3 ;

. Ar' représente un phényle non substitué ou substitué une ou plusieurs fois par un substituant choisi parmi : l'hydrogène, un atome d'halogène, de préférence un atome de chlore ou de fluor, un trifluorométhyle, un alcoxy en $C_1$-$C_4$, un alkyle en $C_1$-$C_4$, lesdits substituants étant identiques ou différents ; un thiényle ; un benzothiényle ; un naphtyle ; un indolyle éventuellement N-substitué par un alkyle en $C_1$-$C_4$ ;

- R représente l'hydrogène ou un alkyle en $C_1$-$C_6$ ;
- T représente un groupe choisi parmi :

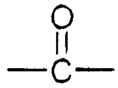 et 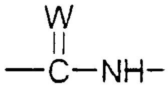

W étant un atome d'oxygène ou de soufre, et
- Z représente un naphtyle, un benzyle, un α-hydroxybenzyle, un phényle, éventuellement substitués par un substituant choisi parmi le chlore ou le fluor, un alkyle en $C_1$-$C_4$ ou un alcoxy en $C_1$-$C_4$.

**2.** Composé selon la revendication 1 de formule (I) dans laquelle R représente hydrogène ou méthyle, Ar' représente un 3,4-dichlorophényle, T est carbonyle, m, Q, J et A $^{\ominus}$ ayant les mêmes significations que dans la revendication 1.

**3.** Iodure de 1-[3-(3,4-dichlorophényl)-4-(N-méthyl-3-isopropoxyphénylacétylamino)butyl]-4-phényl-N(a)-méthyl-1-pipéridinium.

**4.** Iodure de 1-[3-(3,4-dichlorophényl)-4-(N-méthyl-3-isopropoxyphénylacétylamino)butyl]-4-phényl-N(e)-méthyl-1-pipéridinium.

**5.** Iodure de 1-[3-(3,4-dichlorophényl)-4-(N-méthyl-α-hydroxyphénylacétamido)butyl]-4-phényl-N(a)-méthyl-1-pipéridinium.

**6.** Procédé de préparation des composés de formule (I) selon la revendication 1, caractérisé en ce que l'on fait réagir sur le dérivé aminé de formule :

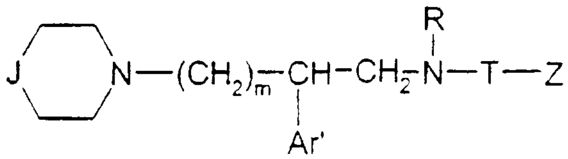 (Ia)

dans laquelle J, m, Ar', R, T et Z sont tels que définis pour (I) dans la revendication 1, un agent alkylant de formule:

A-Q

dans lequel A représente un groupe susceptible de former un anion $A^{\ominus}$ tel que défini pour (I) dans la revendication 1 et Q est tel que défini pour (I) dans la revendication 1 et on chauffe le mélange réactionnel dans un solvant choisi parmi le dichlorométhane, le chloroforme l'acétone ou l'acétonitrile à une température comprise entre la température ambiante et le reflux pendant une à plusieurs heures pour obtenir après traitement selon les méthodes habituelles et après déprotection éventuelle un mélange des conformères axiaux et équatoriaux des sels d'ammonium quaternaires (I) que l'on sépare par recristallisation ou chromatographie.

**7.** Procédé de préparation des composés de formule (I) selon la revendication 1 dans laquelle Q, m, A $^{\ominus}$, Ar', R, T et Z sont tels que définis dans la revendication 1 et dans laquelle J représente un groupe

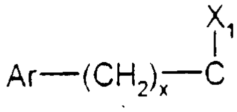

caractérisé en ce que on fait réagir le mésylate de formule :

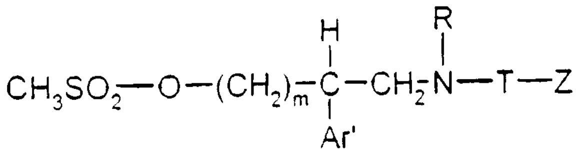

(VI)

dans lequel m, Ar', R, T et Z sont tels que définis dans la revendication 1, avec une amine secondaire de formule :

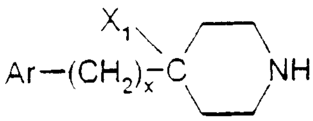

dans laquelle, Ar, x et $X_1$ sont tels que définis dans la revendication 1, puis on transforme le produit (Ia) ainsi obtenu en son sel d'ammonium quaternaire par réaction avec un halogénure d'alkyle A-Q, dans lequel A est choisi parmi le chlore, le brome et l'iode et Q est un $C_1$-$C_6$ alkyle, pour obtenir les composés (I).

**8.** Procédé de préparation des composés de formule (I) selon la revendication 1, caractérisé en ce que l'on fait réagir sur le dérivé de formule :

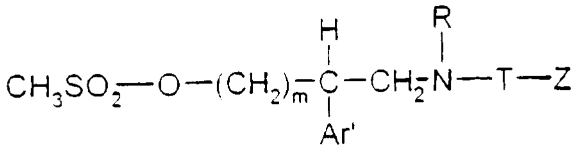

(VI)

dans lequel m, Ar', R, T et Z sont tels que définis dans la revendication 1 une amine tertiaire de formule:

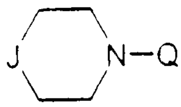

(VII')

dans laquelle J et Q sont tels que définis dans la revendication 1 pour obtenir le composé (I) dans lequel $A^{\ominus}$ représente le méthanesulfonate, cet anion pouvant ensuite être éventuellement échangé par un anion chlorure par élution sur une résine échangeuse d'ions.

**9.** Composition pharmaceutique, caractérisée en ce qu'elle contient au moins un composé selon l'une des revendications 1 à 5 associé à au moins un excipient;

**10.** Composition pharmaceutique selon la revendication 9 sous forme d'unité de dosage.

**11.** Composé de formule :

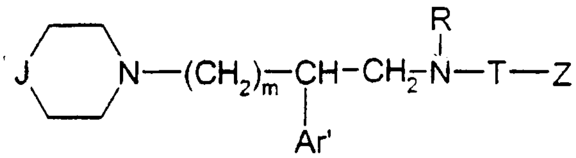

(Ia)

dans laquelle m, Ar', R, T et Z sont tels que définis dans la revendication 1 et J représente un groupe :

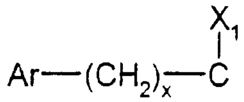

où x est zéro, $X_1$ est l'hydrogène et Ar est tel que défini dans la revendication 1, ou un de ses sels d'addition acides.

**12.** Composé de formule:

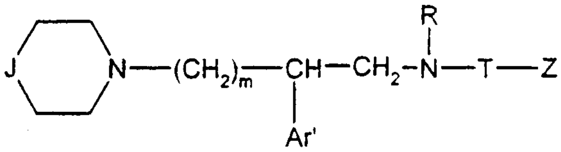

(Ia")

dans laquelle J, m, Ar' et R sont tels que définis dans la revendication 1, T est -C(O)- et Z représente un groupe α-hydroxybenzyle dont le groupe aromatique est non substitué ou substitué une ou plusieurs fois par un chlore, un fluor, un alkyle en $C_1$-$C_4$ ou un alcoxy en $C_1$-$C_4$, ou un de ses sels d'addition acide.

**13.** Composé selon la revendication 11, de formule:

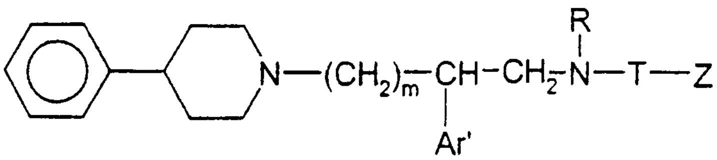

(Ia')

dans laquelle m, Ar', R, T et Z sont tels que définis dans la revendication 1, ou un ses sels d'addition acides.

**14.** Un sel d'addition acide pharmaceutiquement acceptable du composé de formule (Ia') selon la revendication 13.

**15.** Composé selon la revendication 13, caractérisé en ce qu'il est le N-[4-(4-phényl-1-pipéridinyl)-2-(3,4-dichlorophényl)butyl]-N-méthyl-3-isopropoxyphénylacétamide ou un de ses sels pharmaceutiquement acceptables.

**16.** Composé selon la revendication 13, caractérisé en ce qu'il est le chlorhydrate de N-[4-(4-phényl-1-pipéridinyl)-2-(3,4-dichlorophényl)butyl]-N-méthyl-3-isopropoxyphénylacétamide.

**17.** Composé selon la revendication 12, caractérisé en ce qu'il est le le N-[4-(4-phényl-1-pipéridinyl)-2-(3,4-dichlorophényl)butyl]-N-méthyl-α-hydroxy-3-isopropoxyphénylacétamide.

**Claims**

**1.** Quaternary ammonium salt of the formula

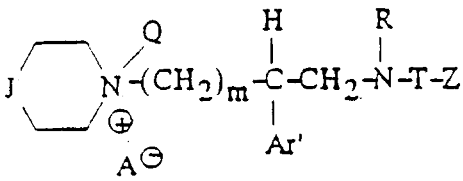

(I)

in which:

- J is

* - either a group

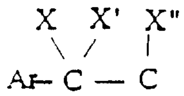

in which:

. Ar is a phenyl which is unsubstituted or monosubstituted or polysubstituted by a substituent selected from hydrogen, a halogen atom, a hydroxyl, a $C_1$-$C_3$ alkoxy, a $C_1$-$C_3$ alkyl, a trifluoromethyl, said substituents being identical or different; a $C_3$-$C_7$ cycloalkyl group; a pyridyl group; and a thienyl group;
. X is hydrogen;
. X' is hydrogen or is joined to X" below to form a carbon-carbon bond, or X and X' together form an oxo group; and
. X" is hydrogen or forms a carbon-carbon bond with X';

* - or a group

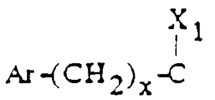

in which:

. Ar is as defined above;
. x is zero or one; and
. $X_1$ is hydrogen, only when x is zero; a hydroxyl; a $C_1$-$C_4$ alkoxy; a $C_1$-$C_4$ acyloxy; a carboxyl; a $C_1$-$C_4$ carbalkoxy; a cyano; a group -NH-CO-Alk, in which Alk is a $C_1$-$C_6$ alkyl; a mercapto group or a $C_1$-$C_4$ alkylthio group;
or $X_1$ forms a double bond with the carbon atom to which it is bonded and with the adjacent carbon atom of the piperidine;

- Q is a $C_1$-$C_6$ alkyl group or a benzyl group;
- $A^{\ominus}$ is an anion selected from chloride, bromide, iodide, acetate, methanesulfonate and paratoluenesulfonate;
- m is 2 or 3;
- Ar' is a phenyl which is unsubstituted or monosubstituted or polysubstituted by a substituent selected from hydrogen, a halogen atom, preferably a chlorine or fluorine atom, a trifluoromethyl, a $C_1$-$C_4$ alkoxy, a $C_1$-$C_4$ alkyl, said substituents being identical or different; a thienyl; a benzothienyl; a naphthyl; and an indolyl optionally N-substituted by a $C_1$-$C_4$ alkyl;
- R is hydrogen or a $C_1$-$C_6$ alkyl;
- T is a group selected from

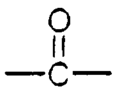 and 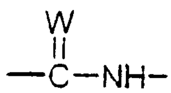

W being an oxygen or sulfur atom; and
- Z is a naphthyl, a benzyl, an α-hydroxybenzyl or a phenyl which are optionally substituted by a substituent selected from chlorine or fluorine, a $C_1$-$C_4$ alkyl and a $C_1$-$C_4$ alkoxy.

**2.** Compound according to claim 1 of formula (I) in which R is hydrogen or methyl, Ar' is a 3,4-dichlorophenyl, T is carbonyl and m, Q, J and $A^{\ominus}$ are as defined in claim 1.

**3.** 1-[3-(3,4-Dichlorophenyl)-4-(N-methyl-3-isopropoxyphenylacetylamino)butyl]-4-phenyl-N(a)-methyl-1-piperidinium iodide.

**4.** 1-[3-(3,4-Dichlorophenyl)-4-(N-methyl-3-isopropoxyphenylacetylamino)butyl]-4-phenyl-N(e)-methyl-1-piperidinium iodide.

**5.** 1-[3-(3,4-Dichlorophenyl)-4-(N-methyl-$\alpha$-hydroxyphenylacetamido)butyl]-4-phenyl-N(a)-methyl-1-piperidinium iodide.

**6.** Method of preparing the compounds of formula (I) according to claim 1, characterized in that an alkylating agent of the formula

A-Q

in which A is a group capable of forming an anion $A^{\ominus}$ as defined for (I) in claim 1 and Q is as defined for (I) in claim 1, is reacted with the amino derivative of the formula

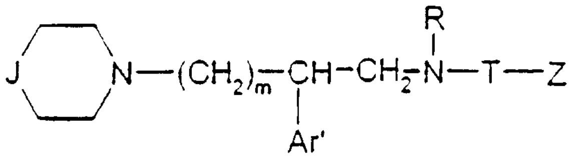 (Ia)

in which J, m, Ar', R, T and Z are as defined for (I) in claim 1, and the reaction mixture is heated in a solvent selected from dichloromethane, chloroform, acetone and acetonitrile, at a temperature between room temperature and the reflux temperature, for one to several hours, to give a mixture of the axial and equatorial conformers of the quaternary ammonium salts (I) after treatment by the customary methods and after deprotection if appropriate, said conformers being separated by recrystallization or chromatography.

**7.** Method of preparing the compounds of formula (I) according to claim 1 in which Q, m, $A^{\ominus}$, Ar', R, T and Z are as defined in claim 1 and in which J is a group

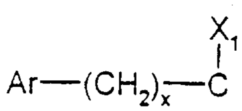

characterized in that the mesylate of the formula

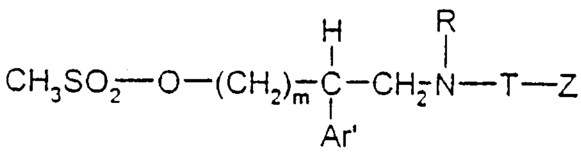 (VI)

in which m, Ar', R, T and Z are as defined in claim 1, is reacted with a secondary amine of the formula

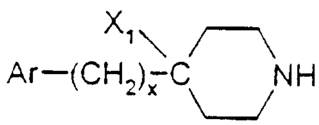

in which Ar, x and $X_1$ are as defined in claim 1, and the resulting product (Ia) is then converted to its quaternary ammonium salt by reaction with an alkyl halide A-Q, in which A is selected from chlorine, bromine and iodine and Q is a $C_1$-$C_6$ alkyl, to give the compounds (I).

**8.** Method of preparing the compounds of formula (I) according to claim 1, characterized in that a tertiary amine of the formula

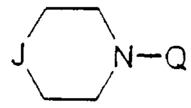 (VII')

in which J and Q are as defined in claim 1, is reacted with the derivative of the formula

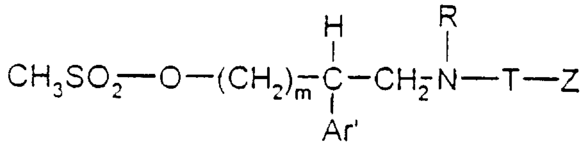 (VI)

in which m, Ar', R, T and Z are as defined in claim 1, to give the compound (I) in which $A^{\ominus}$ is methanesulfonate, it subsequently being possible for this anion to be exchanged, if appropriate, with a chloride anion by elution on an ion exchange resin.

**9.** Pharmaceutical composition, characterized in that it contains at least one compound according to one of claims 1 to 5 in association with at least one excipient.

**10.** Pharmaceutical composition according to claim 9 in the form of a dosage unit.

**11.** Compound of the formula

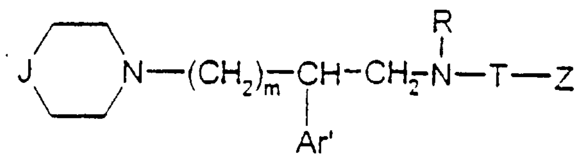 (Ia)

in which m, Ar', R, T and Z are as defined in claim 1 and J is a group

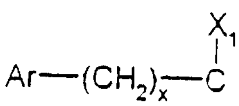

in which x is zero, $X_1$ is hydrogen and Ar is as defined in claim 1, or one of its acid addition salts.

**12.** Compound of

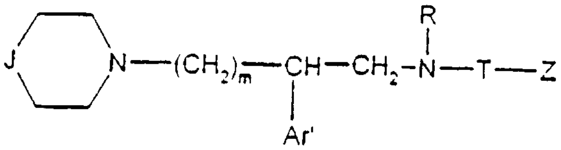

(Ia")

the formula in which J, m Ar' and R are as defined in claim 1, T is -C(O)- and Z is an α-hydroxybenzyl group whose aromatic group is unsubstituted or monosubstituted or polysubstituted by a chlorine, a fluorine, a $C_1$-$C_4$ alkyl or a $C_1$-$C_4$ alkoxy, or one of its acid addition salts.

**13.** Compound according to claim 11 of the formula

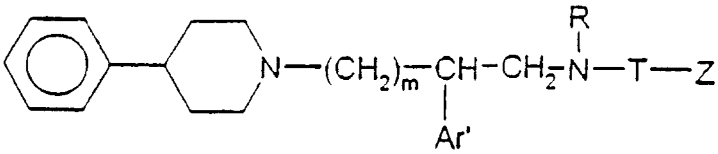

(Ia')

in which m, Ar', R, T and Z are as defined in claim 1, or one of its acid addition salts.

**14.** Pharmaceutically acceptable acid addition salt of the compound of formula (Ia') according to claim 13.

**15.** Compound according to claim 13, characterized in that it is N-[4-(4-phenylpiperidin-1-yl)-2-(3,4-dichlorophenyl)butyl]-N-methyl-3-isopropoxyphenylacetamide or one of its pharmaceutically acceptable salts.

**16.** Compound according to claim 13, characterized in that it is N-[4-(4-phenylpiperidin-1-yl)-2-(3,4-dichlorophenyl)butyl]-N-methyl-3-isopropoxyphenylacetamide hydrochloride.

**17.** Compound according to claim 12, characterized in that it is N-[4-(4-phenylpiperidin-1-yl)-2-(3,4-dichlorophenyl)butyl]-N-methyl-α-hydroxy-3-isopropoxyphenylacetamide.

**Patentansprüche**

**1.** Quartäres Ammoniumsalz der Formel

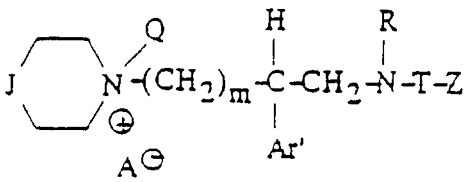

(I),

in der bedeuten:

- J

  * eine Gruppe

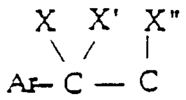,

worin

- Ar nicht substituiertes Phenyl oder Phenyl, das mit einem oder mehreren Substituenten substituiert ist, die unter Wasserstoff, Halogenatomen, Hydroxy, $C_1$-$C_3$-Alkoxy, $C_1$-$C_3$-Alkyl, Trifluormethyl ausgewählt sind, wobei die Substituenten gleich oder verschieden sind, $C_3$-$C_7$-Cycloalkyl, Pyridyl oder Thienyl bedeutet,
- X Wasserstoff darstellt,
- X, Wasserstoff bedeutet oder mit dem weiter unten angegebenen X'' zur Bildung einer Kohlenstoff-Kohlenstoff-Bindung vereinigt ist oder zusammen mit X eine Oxogruppe bildet,
- X'' Wasserstoff darstellt oder mit X' eine Kohlenstoff-Kohlenstoff-Bindung bildet,

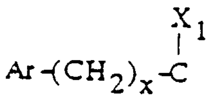,

* oder eine Gruppe worin

- Ar wie weiter oben definiert ist,
- x die Zahl Null oder eins ist,
- $X_1$ Wasserstoff, Hydroxy, $C_1$-$C_4$-Alkoxy, $C_1$-$C_4$-Acyloxy, Carboxy, $C_1$-$C_4$-Carbalkoxy, Cyano, -NH-CO-Alk, worin Alk $C_1$-$C_6$-Alkyl bedeutet, Mercapto, $C_1$-$C_4$-Alkylthio bedeutet, wobei $X_1$ ausschließlich Wasserstoff darstellt, wenn x die Zahl Null ist,
  oder $X_1$ mit dem Kohlenstoffatom, mit dem es verbunden ist, und dem benachbarten Kohlenstoffatom in dem Piperidin eine Doppelbindung bildet,

- Q $C_1$-$C_6$-Alkyl oder Benzyl,
- $A^{\ominus}$ ein Anion, das unter Chlorid, Bromid, Iodid, Acetat, Methansulfonat, p-Toluolsulfonat ausgewählt ist,
- m die Zahl 2 oder 3,
- Ar' nicht substituiertes Phenyl oder Phenyl, das mit einem oder mehreren Substituenten substituiert ist, die unter Wasserstoff, Halogenatomen, vorzugsweise Chlor oder Fluor, Trifluormethyl, $C_1$-$C_4$-Alkoxy, $C_1$-$C_4$-Alkyl ausgewählt sind, wobei die Substituenten gleich oder verschieden sind, Thienyl, Benzothienyl, Naphthyl, Indolyl, das gegebenenfalls am N-Atom mit $C_1$-$C_4$-Alkyl substituiert ist,
- R Wasserstoff oder $C_1$-$C_6$-Alkyl,
- T eine Gruppe, die ausgewählt ist unter

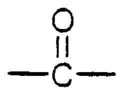 und 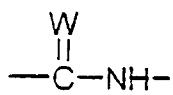,

worin W ein Sauerstoff- oder Schwefelatom ist, und
- Z Naphthyl, Benzyl, α-Hydroxybenzyl, Phenyl, wobei diese Gruppen gegebenenfalls mit einem Substituenten substituiert sind, der unter Chlor, Fluor, $C_1$-$C_4$-Alkyl und $C_1$-$C_4$-Alkoxy ausgewählt ist.

**2.** Verbindung nach Anspruch 1 der Formel (I), worin R Wasserstoff oder Methyl, Ar' 3,4-Dichlorphenyl und T Carbonyl bedeutet und m, Q, J und $A^{\ominus}$ die gleiche Bedeutung wie in Anspruch 1 haben.

**3.** 1-[3-(3,4-Dichlorphenyl)-4-(N-methyl-3-isopropoxyphenylacetylamino)-butyl]-4-phenyl-N(a)-methyl-1-piperidiniumiodid.

**4.** 1-[3-(3,4-Dichlorphenyl)-4-(N-methyl-3-isopropoxyphenylacetylamino)-butyl]-4-phenyl-N(e)-methyl-1-piperidiniumiodid.

**5.** 1-[3-(3,4-Dichlorphenyl)-4-(N-methyl-α-hydroxyphenylacetamido)-butyl]-4-phenyl-N(a)-methyl-1-piperidiniumiodid.

**6.** Verfahren zur Herstellung der Verbindungen der Formel (I) nach Anspruch 1, dadurch gekennzeichnet, daß das Aminderivat der Formel

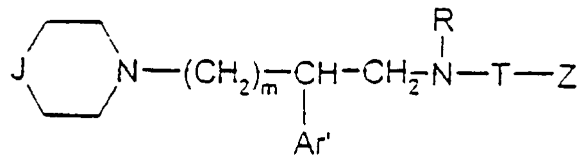 (Ia),

in der J, m, Ar', R, T und Z wie für Formel (I) in Anspruch 1 definiert sind, mit einem Alkylierungsmittel der Formel

A-Q

umgesetzt wird, worin A eine Gruppe darstellt, die ein Anion $A^{\ominus}$ zu bilden vermag, das wie für Formel (I) in Anspruch 1 definiert ist, und Q wie für Formel (I) in Anspruch 1 definiert ist, daß das Reaktionsgemisch eine bis mehrere Stunden in einem Lösungsmittel, das unter Dichlormethan, Chloroform, Aceton und Acetonitril ausgewählt wird, auf eine Temperatur erwärmt wird, die im Bereich von Umgebungstemperatur bis zur Rückflußtemperatur liegt, um nach einer Behandlung nach herkömmlichen Verfahren und gegebenenfalls nach der Entfernung von Schutzgruppen ein Gemisch des axialen und des äquatorialen Konformers der quartären Ammoniumsalze (I) zu erhalten, das durch Umkristallisieren oder chromatographisch getrennt wird.

**7.** Verfahren zur Herstellung der Verbindungen der Formel (I) nach Anspruch 1, worin Q, m, $A^{\ominus}$, Ar', R, T und Z wie in Anspruch 1 definiert sind und J eine Gruppe

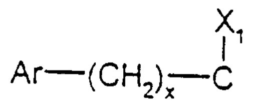

darstellt, dadurch gekennzeichnet, daß das Mesylat der Formel

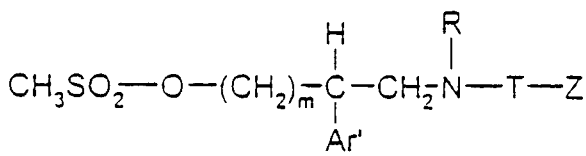 (VI),

worin m, Ar', R, T und Z wie in Anspruch 1 definiert sind, mit einem sekundären Amin der Formel

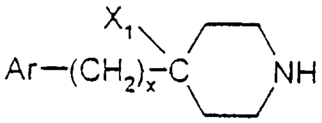

,

worin Ar, x und $W_1$ wie in Anspruch 1 definiert sind, umgesetzt wird, und daß das so erhaltene Produkt (Ia) durch Umsetzung mit einem Alkylhalogenid A-Q, worin A unter Chlor, Brom und Iod ausgewählt wird und Q ein $C_1$-$C_6$-Alkyl ist, in das quartäre Ammoniumsalz umgewandelt wird, um die Verbindungen (I) zu erhalten.

**8.** Verfahren zur Herstellung der Verbindungen der Formel (I) nach Anspruch 1, dadurch gekennzeichnet, daß das Derivat der Formel

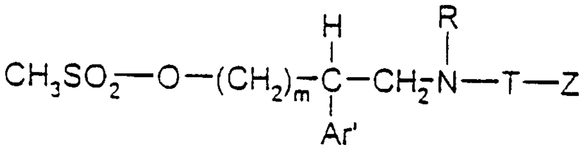

(VI),

in der m, Ar', R, T und Z wie in Anspruch 1 definiert sind, mit einem tertiären Amin der Formel

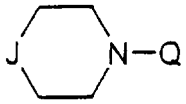

(VII'),

worin J und Q wie in Anspruch 1 definiert sind, umgesetzt wird, um die Verbindung (I) zu erhalten, in der $A^{\ominus}$ Methansulfonat bedeutet, wobei dieses Anion anschließend gegebenenfalls durch Elution auf einem Ionenaustauscherharz gegen Chlorid ausgetauscht werden kann.

**9.** Pharmazeutische Zusammensetzung, dadurch gekennzeichnet, daß sie mindestens eine Verbindung nach einem der Ansprüche 1 bis 5 in Kombination mit mindestens einem Exzipiens enthält.

**10.** Pharmazeutische Zusammensetzung nach Anspruch 9 in Form einer Dosiereinheit.

**11.** Verbindung der Formel

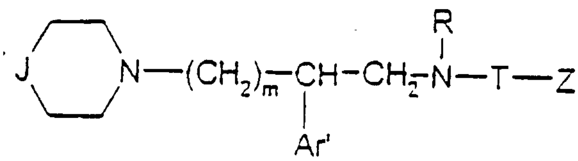

(Ia),

worin m, Ar', R, T und Z wie in Anspruch 1 definiert sind und J eine Gruppe

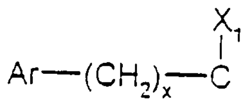

bedeutet, in der x Null ist, $X_1$ Wasserstoff bedeutet und Ar wie in Anspruch 1 definiert ist, oder eines ihrer Säureadditionssalze.

**12.** Verbindung der Formel

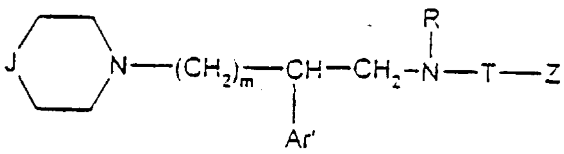

(Ia"),

in der J, m, Ar' und R wie in Anspruch 1 definiert sind, T -C(O)- bedeutet und Z α-Hydroxybenzyl darstellt, dessen aromatische Gruppe nicht substituiert ist oder einfach oder mehrfach mit Chlor, Fluor, $C_1$-$C_4$-Alkyl oder $C_1$-$C_4$-Alkoxy substituiert ist, oder eines ihrer Säureadditionssalze.

**13.** Verbindung nach Anspruch 11 der Formel

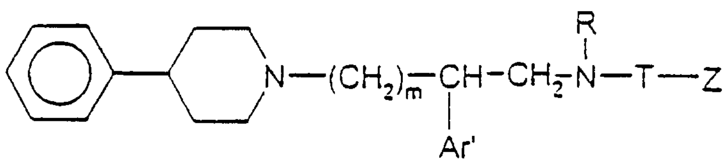

(Ia'),

worin m, Ar', R, T und Z wie in Anspruch 1 definiert sind, oder eines ihrer Säureadditionssalze.

**14.** Pharmazeutisch akzeptables Säureadditionssalz der Verbindung der Formel (Ia') nach Anspruch 13.

**15.** Verbindung nach Anspruch 13, dadurch gekennzeichnet, daß es sich um N-[4-(4-Phenyl-1-piperidinyl)-2-(3,4-dichlorphenyl)butyl]-N-methyl-3-isopropoxyphenylacetamid oder eines seiner pharmazeutisch akzeptablen Salze handelt.

**16.** Verbindung nach Anspruch 13, dadurch gekennzeichnet, daß es sich um das Hydrochlorid von N-[4-(4-Phenyl-1-piperidinyl)-2-(3,4-dichlorphenyl)-butyl]-N-methyl-3-isopropoxyphenylacetamid handelt.

**17.** Verbindung nach Anspruch 12, dadurch gekennzeichnet, daß es sich um N-[4-(4-Phenyl-1-piperidinyl)-2-(3,4-dichlorphenyl)butyl]-N-methyl-α-hydroxy-3-isopropoxyphenylacetamid handelt.